# EUROPEAN PATENT APPLICATION

(11) **EP 4 026 910 A1**
(43) Date of publication of application: **13.07.2022**
(21) Application number: 20861118.6
(22) Date of filing: 04.09.2020
(51) Int. Cl.: C12N 15/90, C12N 5/10, A61K 35/28, A61P 35/00, A61K 38/17, A61K 48/00

(54) **METHOD FOR EVALUATING GENE EDITING THERAPY BASED ON OFF-TARGET ASSESSMENT**

(30) Priority: 04.09.2019 WO PCT/CN2019/104303
(71) Applicant: Edigene Inc., Beijing 102206 (CN)
(72) Inventor: YUAN, Pengfei, Beijing 102206 (CN); FANG, Riguo, Beijing 102206 (CN); JIN, Ming, Beijing 102206 (CN); ZHANG, Yongjian, Beijing 102206 (CN)
(74) Representative: Lavoix
(86) International application number: PCT/CN2020/113562
(87) International publication number: WO 2021/043278

(57) **Abstract**

Disclosed is a method for determining the suitability of a cell therapy for an individual. By means of using a bioinformatic method and an experimental analysis method for an unbiased potential off-target site, a potential off-target site of a genetically modified cell from an individual is predicted before administering a cell therapy to an individual, and the potential off-target site is monitored after administering, to the individual, the genetically modified cell from the individual to determine whether gene editing occurs at the potential off-target site and to evaluate the suitability of the therapy on the basis of the occurrence of a gene editing event.

## Description

### FIELD OF THE INVENTION

The present invention belongs to the field of gene therapy, and particularly relates to a method for determining the suitability of a cell therapy for an individual.

### BACKGROUND OF THE INVENTION

Thalassemia is a group of inherited hemolytic anemias, including 4 types such as α type, β type, δβ type and δ type, wherein both α- and β-thalassemias are relatively common.

The human β-globin gene cluster consists of five functional genes arranged in chromosome 11 in the same order in which they are expressed during development: ε-, Gγ-, Aγ-, δ-, and β-globin genes. Fetal hemoglobin (HbF) is the dominant form of hemoglobin present in the fetus where γ-globin genes are high expressed; Later there is a switch from predominant expression of HbF to adult hemoglobin (HbA), with s the γ-globin genes expression being gradually turned off and the β-globin genes expression switched on. β-thalassemia involves mutations in the β-globin gene, and the mutations prevents erythrocytes from producing enough oxygen-carrying hemoglobin (the adult hemoglobin), thereby causing anemia.

BCL11A is known to be a gene related to the expression of the fetal hemoglobin (HbF). The protein product of the BCL11A gene is a trans-acting factor that inhibits transcription of the β-globin gene. Decreased expression of the BCL11A gene is related to continuous expression of the fetal hemoglobin (HbF).

Researches have shown that, the inactivated BCL11A gene may enable the erythrocytes to continuously produce hemoglobin of a fetal form after birth, and the produced hemoglobin may replace defective adult hemoglobin (HbA). A genetic enhancer of the BCL11A has activity in the erythrocytes only. Therefore, the BCL11A enhancer may be edited in almost all hematopoietic stem cells; and the expression of the BCL11A is decreased by cutting the BCL11A gene (which inhibits production of the fetal hemoglobin), thereby removing an inhibiting effect on the expression of the fetal hemoglobin gene HBG, increasing total hemoglobin in blood of patients with thalassemia and achieving the aim of treating diseases.

At present, the BCL11A gene is edited based on a CRISPR-Cas9 gene editing technology in natural medicine research. The patients with thalassemia are treated by a cell therapy. Hematopoietic stem cells are taken out of bodies of the patients and then edited. The edited cells are differentiated into the erythrocytes so as to produce functional hemoglobin; and then original hematopoietic stem cells of the patients are replaced with the edited cells.

For example, autologous CD34+ cell injection of the BCL11A enhancer is edited by CRISPR/Cas9. Based on the CRISPR/Cas9 gene editing technology, the expression of the BCL11A is decreased by editing a specific target of an erythroid-specific enhancer of the BCL11A gene in autologous CD34+ hematopoietic stem cells of the patients, thereby removing the inhibiting effect on the expression of the fetal hemoglobin gene HBG, increasing the total hemoglobin in blood of patients with thalassemia and achieving the aim of treating diseases.

However, one of the problems existing during CRISPR/Cas9 gene editing is an off-target effect. Mismatch of a certain probability may exist during binding of sgRNA and genomic DNA sequences, thereby causing off-target mutation at the off-target site.

Therefore, a method that may monitor a potential off-target site after administering a cell therapy to an individual, can determine whether gene editing occurs at the potential off-target site and assess the suitability of the therapy to the individual on the basis of the occurrence of a gene editing event.

### SUMMARY OF THE INVENTION

In the present invention, by using a bioinformatic prediction analysis method and an unbiased potential off-target site experiment analysis method, a potential off-target site of a genetically modified cell from an individual is detected before administering a cell therapy to the individual. Through the potential off-target site detected by the method in the present invention, an off-target tagged site mode is constructed; and after the cell therapy is administered to the individual, the potential off-target site is tracked and monitored, so as to timely understand the occurrence of the off-target effect in the patients , and assess the suitability of the therapy to the individual, the effect of the gene editing cell therapy, and whether the cell therapy needs intervention, thereby partially achieving an aim of effectively treating the diseases while lowering risk of the cell therapy.

In one aspect, the present invention relates to a method for determining the suitability of a cell therapy for an individual. Specifically, the present invention provides a method for determining the suitability of the cell therapy for the individual, including a step of administering a genetically modified cell population from an individual to the individual, wherein the genetically modified cells are modified at a target site through gene editing. The method includes the following steps:
a) determining the occurrence of gene editing of each site in multiple off-target tagged sites in the genetically modified cells or offspring thereof; and
b) assessing the suitability of the therapy based on the occurrence of gene editing,
wherein the nonoccurrence of gene editing at any of the multiple off-target tagged sites indicates that the cell therapy is suitable for the individual.

In another aspect, the present invention further provides a method for determining unsuitability of a cell therapy for an individual. The method includes a step of administering a genetically modified cell population from an individual to the individual, wherein the genetically modified cells are modified at a target site through gene editing. The method includes the following steps:
a) determining the occurrence of gene editing of each site in multiple off-target tagged sites in the genetically modified cells or offspring thereof; and
b) assessing the suitability of the therapy based on the occurrence of gene editing,
wherein the occurrence of gene editing at any of the multiple off-target tagged sites indicates that the cell therapy is unsuitable for the individual.

In one aspect, the present invention relates to a method for treating an individual. Specifically, the present invention provides a cell therapy. The cell therapy includes: 1) a step of determining suitability of the cell therapy for an individual; and 2) a step of administering a genetically modified cell population from an individual to the individual suitable for the cell therapy, wherein the genetically modified cells are modified at a target site through gene editing. The step of determining the suitability of the cell therapy for the individual includes:
a) determining the occurrence of gene editing of each site in multiple off-target tagged sites in the genetically modified cells or offspring thereof; and
b) assessing the suitability of the therapy based on the occurrence of gene editing,
wherein the nonoccurrence of gene editing at any of the multiple off-target tagged sites indicates that the cell therapy is suitable for the individual.

In one aspect, the present invention provides a method for diagnosing whether off-target occurs accompanying with an individual gene editing cell therapy. The method includes: a) administering a genetically modified cell population from an individual to the individual, wherein the genetically modified cells are modified at a target site through gene editing; and
b) determining the occurrence of gene editing of each site in multiple off-target tagged sites in the genetically modified cells or offspring thereof,
wherein the nonoccurrence of gene editing at any of the multiple off-target tagged sites indicates that the cell therapy has no off-target.

In one aspect, the present invention provides a method for monitoring an effect of a gene editing cell therapy. The method includes: a) administering a genetically modified cell population from an individual to the individual, wherein the genetically modified cells are modified at a target site through gene editing; and
b) determining the occurrence of gene editing of each site in multiple off-target tagged sites in the genetically modified cells or offspring thereof,
wherein the nonoccurrence of gene editing at any of the multiple off-target tagged sites indicates that the cell therapy has good treatment effect on the individual.

In one aspect, the present invention provides a method for monitoring quality of a genetically modified cell product from a certain individual. The method includes: a) determining the occurrence of gene editing of each site in multiple off-target tagged sites in the genetically modified cells,
wherein the nonoccurrence of gene editing at any of the multiple off-target tagged sites indicates that the cell product is suitable for the individual for conducting cell therapy. In one aspect, the present invention provides a method for judging whether an intervention therapy needs to be administered to an individual of the gene editing cell therapy. The method includes: a) administering a genetically modified cell population from an individual to the individual, wherein the genetically modified cells are modified at a target site through gene editing; and
b) determining the occurrence of gene editing of each site in multiple off-target tagged sites in the genetically modified cells or offspring thereof,
wherein the occurrence of gene editing at any of the multiple off-target tagged sites indicates a need for administration of an intervention therapy to the individual with gene editing cell therapy.

In some embodiments of the above method, the method includes conducting the determination step on the genetically modified cell before administering the genetically modified cell to the individual. In some embodiments of the above method, the method further includes administration of the genetically modified cell to the individual. In some embodiments of the above method, the method includes conducting the determination step on the offspring of the genetically modified cell after administration of the genetically modified cells. In some embodiments of the above method, the determination step is conducted within about at least one month after the administration of the genetically modified cell, such as at least 30 days, 40 days, 50 days and 60 days. In some embodiments of the above method, the method further includes repeating the determination and assessment steps once or more times, such as 2 times, three times, four times, five times or more times. For example, the determination and assessment steps are conducted about every month, about every two months, about every three months, about every four months, about every five months and about every six months. In some embodiments of the above method, the determination and assessment steps are repeated at a frequency of about once every month to about once every year.

In some embodiments of the above method, the method further includes treatment of the individual with the intervention therapy after the assessment step. In some embodiments of the above method, the intervention therapy is chemotherapy. In some embodiments of the above method, the intervention therapy is removal of the administered genetically modified cell population. In some embodiments of the above method, the intervention therapy is administration of a second genetically modified cell population from the individual.

In some embodiments of the above method, the multiple off-target tagged sites include at least about 10 off-target tagged sites (such as 10-50, 10-45, 10-40, 10-35, 10-30, 10-25, 10-20 and 10-15).

In some embodiments of the above method, there is less than 0.1% of gene editing efficiency at the off-target tagged sites; or, compared with control cells that are not subjected to gene editing modification, the gene editing efficiency of the genetically modified cell at the off-target tagged sites is less than 2 times that of the gene editing efficiency of a control group at the off-target tagged sites, which indicates that no gene editing occurs at the off-target tagged sites.

In one aspect, the present invention relates to a method for assessing off-target gene editing in a genetically modified cell population or offspring thereof. The genetically modified cells are modified at the target sites through gene editing. The method includes determination of occurrence of gene editing of each site in the multiple off-target tagged sites. The multiple off-target tagged sites are obtained by manners as follows: 1) identifying first multiple off-target tagged sites based on their sequence similarity with the target sites; and/or 2) identifying second multiple off-target tagged sites by using an in-vitro test method.

In some embodiments of the above method, the determination step is conducted through DNA sequencing. In some embodiments of the above method, the determination step includes: 1) amplifying nucleic acids comprising the multiple off-target tagged sites by using multiple primer sets; and 2) conducting sequencing analysis on the amplified nucleic acids.

The present invention relates to a method for obtaining multiple off-target tagged sites in a genetically modified cell population. The genetically modified cells are modified at the target sites through gene editing. The method includes: 1) identifying first multiple off-target tagged sites based on their sequence similarity with the target sites; and/or 2) identifying second multiple off-target tagged sites by using an in-vitro test method.

In some embodiments of the above method, the in-vitro test method includes any genome-wide unbiased off-target analysis. In some embodiments of the above method, the method includes any one or more of the followings: BLESS, GUIDE-seq, HIGTS, Circle-seq, SITE-seq and Digenome-seq. In some embodiments of the above method, the in-vitro test method includes the Digenome-seq. In some embodiments of the above method, the in-vitro test method is conducted under a saturation condition. In some embodiments of the above method, the genome of a group of cells is extracted by the in-vitro test method, and then gene editing is conducted under a saturation condition. In some embodiments of the above method, the saturation condition allows occurrence of effective cutting in at least more than 90% of the target sites in the genome of the group of cells, e.g., the effective cutting occurs in at least 91%, 92%, 93%, 94%, 95%, 96%, 97%, 98%, 99% or 100% of the target sites.

In some embodiments of the above method, the genetically modified cells are modified by the CRISPR/Cas system. In some embodiments of the above method, the target sites are located at BCL11A gene loci.

The present invention relates to a kit for assessing off-target editing of a genetically modified cell population or offspring thereof. The genetically modified cells are modified at the target sites through gene editing . The kit comprises: 1) one or more components of a gene editing system, used for producing the genetically modified cell; and 2) multiple primer sets used for amplifying nucleic acids comprising the multiple off-target tagged sites.

In some embodiments of the above method, the multiple off-target tagged sites are obtained by manners as follows: 1) identifying first multiple off-target tagged sites based on their sequence similarity with the target sites; and/or 2) identifying second multiple off-target tagged sites by using an in-vitro test method. In some embodiments, the gene editing system is a CRISPR/Cas9 system. In some embodiments, the kit for assessing off-target editing of the genetically modified cell population or offspring thereof further includes one or more primer sets. The primer sets are used for amplifying nucleic acids comprising the target sites.

In some embodiments of the above method and kit, the gene editing method or system is selected from ZFN, TALEN, CRISPR or any other gene editing method or system. In some embodiments of the above method and kit, the gene editing method or system is a CRISPR gene editing method or system. In some embodiments of the above method and kit, the target sites are BCL11A sites in CD34 positive hematopoietic stem cells/progenitor cells. In some embodiments of the above method and kit, the target sites are located at a nucleotide sequence between exon 2 and exon 3 of BCL11A enhancer genes, e.g., located at or corresponding to sites at locations such as +55:Chr2:60497676-60498941, +58:Chr2:60494251-60495546 and +62:Chr2:60490409-60491734 recorded in hg38. In some embodiments of the above kit, the kit comprises sgRNA targeting the above target sites, or includes a nucleotide sequence of the sgRNA, or a nucleotide sequence that encodes the sgRNA. In some embodiments of the above kit, the kit comprises any one or more sgRNA selected from SEQ ID NOs: 3-25. In some embodiments of the above kit, the kit comprises a Cas9 protein or an expression vector or a nucleotide sequence that encodes the Cas9 protein. In some embodiments of the above kit, the one or more primer sets comprise one or more sequences: SEQ ID NOs: 26-117.

### DESCRIPTION OF DRAWINGS OF THE INVENTION

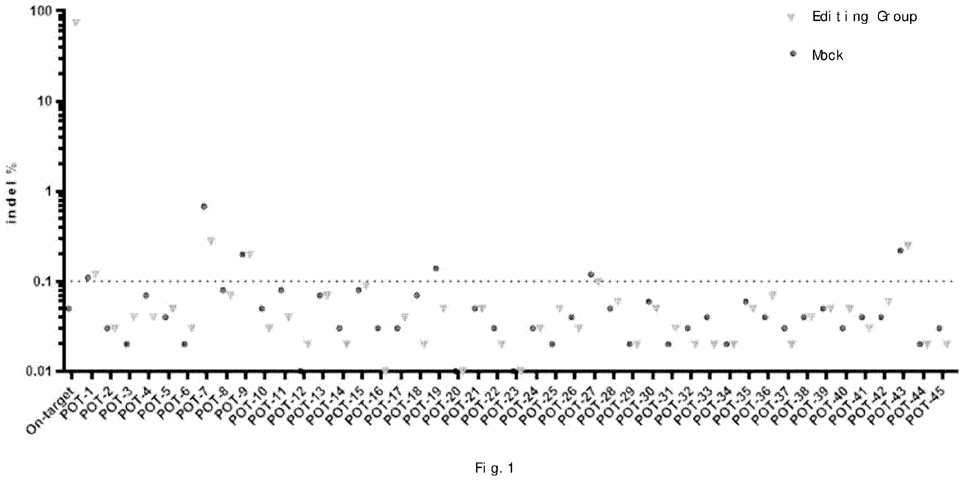

Fig. 1 shows sgRNA potential off-target site analysis for a BCL11A erythroid-specific enhancer. The horizontal axis is the each site; and the vertical axis is gene editing efficiency (indel). All results may be divided into three groups: 1) a target site group, that is, the group of which the horizontal coordinate is On-target; 2) an editing group with a gene editing efficiency of more than 0.1%, but having no significant difference from a control group (Mock) (not more than 2 times that of the Mock group), such as POT-1, POT-7, POT-9 and POT-43; and 3) an editing group with gene editing efficiency of less than 0.1%, i.e., horizontal axis sites except for potential off-target sites comprised in the groups 1) and 2). indel: (insertion and deletion).

### DETAILED DESCRIPTION OF THE INVENTION

In one aspect, the present invention relates to a method for determining the suitability of a cell therapy for an individual. Specifically, the present invention provides the method for determining the suitability of the cell therapy for the individual. The method includes a step of administering a genetically modified cell population from an individual to the individual, wherein the genetically modified cells are modified at a target site through gene editing. The method includes the following steps:
a) determining the occurrence of gene editing of each site in multiple off-target tagged sites in the genetically modified cells or offspring thereof; and
b) assessing the suitability of the therapy based on the occurrence,
wherein the nonoccurrence of gene editing at any of the multiple off-target tagged sites indicates that the cell therapy is suitable for the individual.

In another aspect, the present invention further provides a method for determining unsuitability of a cell therapy for an individual. The method includes a step of administering a genetically modified cell population from an individual to the individual, wherein the genetically modified cells are modified at a target site through gene editing. The method includes the following steps:
a) determining the occurrence of gene editing of each site in multiple off-target tagged sites in the genetically modified cells or offspring thereof; and
b) assessing the suitability of the therapy based on the occurrence,
wherein the occurrence of gene editing at any of the multiple off-target tagged sites indicates that the cell therapy is unsuitable for the individual.

In some embodiments, the genetically modified cells are cells modified by the CRISPR/Cas system. In some embodiments, the genetically modified cells are CD34 positive hematopoietic stem cells/progenitor cells. In some embodiments, the target sites are located at BCL11A gene loci. In some embodiments, the target sites are located at BCL11A gene loci of the CD34 positive hematopoietic stem cells/progenitor cells.

A term "cell therapy" used in the present invention is a therapeutic method for transplanting living cells, such as healthy living cells and modified living cells, into an individual to supplement, offset, replace or repair pathological cells so as to improve structures and functions of cells and tissues.

The genetically "modified" cells refer to cells with gene level changes. For example, the cells may be modified on a molecular level through a gene editing method, so that the cells are changed on a gene level. The genetically modified cell may be changed on a cell level, e.g., protein expression is enhanced or inhibited, and cell functions are increased or decreased. For example, a BCL11A erythroid-specific enhancer is subjected to gene editing through a gene editing method; and BCL11A gene expression is down-regulated, thereby removing inhibition of the BCL11A gene on expressions of the γ-globin and HbF, increasing the expressions of the γ-globin and HbF in the erythrocytes and treating β-thalassemia and sivklecell anemia. In specific embodiments, in isolated CD34 positive hematopoietic stem cells/progenitor cells ("CD34 positive HSPC"), the BCL11A gene is disrupted through a gene editing method, such as a gene editing technology selected from ZFN, TALEN or CRISPR; and then the genetically modified cells with decreased BCL11A functions are produced.

Gene modification may be realized through gene editing or regulation of a DNA level. For example, the cells may be subjected to gene modification through any or a combination of the following methods: zinc finger nuclease (ZFN), transcription activator-like effector nuclease (TALEN) and clustered regularly interspaced short palindromic repeat (CRISPR) technologies.

The "CRISPR" or "CRISPR/Cas" technology used in the present application is a gene editing technology, and includes but not limited to each CRISPR/Cas system that naturally occurs or is artificially designed, such as a CRISPR/Cas9 system. The naturally occurring CRISPR/Cas system is an adaptive immune defence of bacteria and archaebacteria in long-term evolution and may be used for resisting invading viruses and exogenous DNA. For example, an operating principle of the CRISPR/Cas9 is as follows: crRNA (CRISPR-derived RNA) and tracrRNA (trans-activating RNA) are bound to form a tracrRNA/crRNA complex through base pairing. The complex guides a nuclease Cas9 protein to cut double-stranded DNA at sequence target sites paired with the crRNA. However, by artificially designing the tracrRNA and crRNA, sgRNA (single guide RNA, or guide RNA) with a guide effect may be modified and formed, and is enough to guide site-specific cutting of the Cas9 on the DNA. As a RNA-guided dsDNA binding protein, the Cas9 effector nuclease can co-localize the RNA, DNA and protein so as to own huge modification potential. The CRISPR/Cas system may use Cas proteins of type I, II or III. In some implementation modes of the present invention, the Cas9 is used in the method. Other applicable CRISPR/Cas systems include but not limited to systems and methods described in WO2013176772, WO2014065596, WO2014018423, US8,697,359, PCT/CN2018/112068 and PCT/CN2018/112027.

In some embodiments of the above method, the method includes gene modification of the cell. In some embodiments of the above method, the gene modification includes "CRISPR" or "CRISPR/Cas" gene editing technology.

The "individual" in the present application refers to a patient or a subject who plans to be treated or is being treated or has been treated with gene-edited autologous cells, wherein the patient or subject includes a β thalassemia patient or a sicklecell anemia patient.

The "off-target" or "off-target effect" in the present application refers to a phenomenon that modification occurs departing from a preset target site. For a CRISPR/Cas gene editing method, since the CRISPR system is recognized by virtue of complementary base pairing of the sgRNA and chromosome, the higher the sequence similarity of untargeted sites and target sites is, the higher possibility of the off-target effect is. It is widely believed that, possible side effects of the gene therapy/cell therapy, such as occurrence of cancers, may be greatly related to the off-target effect; and gene loci that shall not be edited are edited. Therefore, predicting gene editing off-target sites and monitoring the sites are crucially important to lower risk of the gene therapy/cell therapy.

The "target site" in the present application is a preset sequence that is modified by using gene modification related methods such as the gene editing method. For example, in a gene editing method using the CRISPR, the sgRNA may be bound with the target site in a complementary base pairing manner and guide the Cas protein to conduct gene modification on the target site. The "untargeted site" in the present application refers to any other sequence segment in genome of the target site except for the target site. The "off-target site" in the present application refers to any untargeted site subjected to gene modification by using the gene editing method. The "off-target tagged site" in the present application refers to any untargeted site with off-target risk in the genome. The off-target tagged sites and the target site have high sequence similarity; and due to the similarity, a nucleotide sequence of the off-target tagged site is easily changed in the gene modification process for the target site, thereby achieving the off-target effect at the off-target tagged site and forming the off-target site. The "off-target tagged sites" in the present application includes first multiple off-target tagged sites and second multiple off-target tagged sites. In the present application, unless otherwise specified, the "off-target tagged site" and "potential off-target site" may be interchangeable.

The "first multiple off-target tagged sites" refer to a group of off-target tagged sites identified based on their sequence similarity with the the target site through a bioinformatic method. In some embodiments of the present application, the first multiple off-target tagged sites may be identified through the sequence similarity. For example, for the CRISPR/Cas gene editing method, since the CRISPR system is recognized by virtue of complementary base pairing of the sgRNA and chromosome, it may be believed that, the higher the sequence similarity is, the higher possibility of the off-target effect is. In the CRISPR system, the target site is generally composed of an region bound with the sgRNA (generally 19-21 bp, such as 20 bp) and a PAM region. In some embodiments, sites with the sequence similarity (or called sequence identity) of more than 80% (such as 85%, 90%, 95%, 96%, 97%, 98% and 99%) with the target site are identified as the off-target tagged sites. The "sequence similarity" or the sequence identity refers to a similarity level between two nucleotide sequences. The sequence similarity may be defined as a percentage of the same nucleotide residues between a target nucleotide sequence and a reference nucleotide sequence. The nucleotide sequence similarity may be determined by using multiple known methods in the art, such as publically available computer software, i.e., BLAST, BLAST-2, ALIGN or Megalign (DNASTAR) software. Appropriate parameters used for sequence alignment can be determined by those skilled in the art and include any algorithm that meets maximum alignment requirements of the compared sequences.

The "second multiple off-target tagged sites" in the present application refer to a group of off-target tagged sites determined by identifying modification occurred after actual editing by using an in-vitro test method. In some embodiments, the in-vitro test method includes any genome-wide unbiased off-target analysis. In some embodiments of the above method, the method includes any or multiple of the followings: BLESS, GUIDE-seq, HIGTS, Circle-seq, SITE-seq and Digenome-seq. The BLESS is to ligate double-strand broken DNA ends with biotin, then perform enriching and sequencing on the DNA ends with streptavidin and analyze the DNA ends [Yan WX, Mirzazadeh R, Garnerone S, Scott D, Schneider MW, et al. 2017. BLISS is a versatile and quantitative method for genome-wide profiling of DNA double-strand breaks. Nat. Commun. 8:150583]. The GUIDE-seq is to insert dsODN (double-stranded oligodeoxynucleotide) into a DNA double-strand break region for tagging DSB and performing sequencing and analysis [Tsai SQ, Zheng Z, Nguyen NT, Liebers M, Topkar VV, et al. 2015. GUIDE-seq enables genome-wide profiling of off-target cleavage by CRISPR-Cas nucleases. Nat. Biotechnol. 33:187-97]. The HTGTS is to ligate two double-strand break regions for tagging with translocation and to perform sequencing and analysis [Frock RL, Hu J, Meyers RM, Ho YJ, Kii E, Alt FW. 2015. Genome-wide detection of DNA double stranded breaks induced by engineered nucleases. Nat. Biotechnol. 33:179-86]. The Circle-seq, SITE-seq and Digenome-seq are to tag cut sites in vitro and then to perform sequencing and analysis [6. Tsai SQ, Nguyen NT, Malagon-Lopez J, Topkar VV, Aryee MJ, Joung JK. 2017. CIRCLE-seq: a highly sensitive in vitro screen for genome-wide CRISPR-Cas9 nuclease off-targets. Nat. Methods 14:607-14; Cameron P, Fuller CK, Donohoue PD, Jones BN, Thompson MS, et al. 2017. Mapping the genomic landscape of CRISPR-Cas9 cleavage. Nat. Methods 14:600-6 ; Kim D, Bae S, Park J, Kim E, Kim S, et al. 2015. Digenome-seq: genome-wide profiling of CRISPRCas9 off-target effects in human cells. Nat. Methods 12:237-43]. For the above several methods, the produced DNA double-strand breaks are cut by detecting Cas protein cleavage by directly utilizing high-throughput sequencing so as to discover the off-target sites; and low-frequency off-target sites can be effectively discovered.

In the present application, the "first multiple off-target tagged sites" and the "second multiple off-target tagged sites" may have intersection or may have no intersection.

The "occurrence of gene editing" in the present application refers to a phenomenon that specific or nonspecific gene modification occurs at the target site or the off-target tagged site due to the gene editing method.

The present application provides a method for assessing an off-target effect in a genetically modified cell population or offspring thereof. The genetically modified cells are modified at the target sites through gene editing. The method includes determination of occurrence of gene editing of each site in the multiple off-target tagged sites. The multiple off-target tagged sites are obtained by manners as follows: 1) identifying the first multiple off-target tagged sites based on their sequence similarity with the target sites; and/or 2) identifying the second multiple off-target tagged sites by using the in-vitro test method. In some embodiments, the determination step is conducted by DNA sequencing. In some embodiments, the determination step includes: 1) amplifying nucleic acids comprising the multiple off-target tagged sites by using multiple primer sets; and 2) conducting sequencing analysis on the amplified nucleic acids. The present application further provides a method for obtaining multiple off-target tagged sites in a genetically modified cell population. The genetically modified cells are modified at the target sites through gene editing. The method includes: 1) identifying first multiple off-target tagged sites based on their sequence similarity with the target sites; and/or 2) identifying second multiple off-target tagged sites by using the in-vitro test method. In some embodiments, the in-vitro test method includes any one or more of the followings: BLESS, GUIDE-seq, HIGTS, Circle-seq, SITE-seq and Digenome-seq.

In some embodiments, the above in-vitro test method is conducted under a saturation condition. The term "saturation condition" refers to a condition that more than 90% of effective cutting occurs at the target site in vitro. In some embodiments, genome of a group of cells is extracted by the in-vitro test method, and then gene editing is conducted under the saturation condition. In some embodiments, the saturation condition allows occurrence of effective cutting in at least 90% or more of the target sites in the genome of the group of cells, e.g., the effective cutting occurs in at least 91%, 92%, 93%, 94%, 95%, 96%, 97%, 98%, 99% or 100% of the target sites.

During sequencing, such as deep sequencing, an error of a certain ratio (occurring in a PCR process) may be produced; and the error is related to the sites (e.g., some sites are unfavorable for the PCR, and more errors may occur in the PCR process). Generally speaking, for most of the sites, a detection limit of a deep sequencing detection method is about 0.1% (Nat Rev Genet. 2018 May; 19(5): 269-285, Next Gener Seq Appl. 2014; 1: 1000106). In other words, if only about 0.1% or less than about 0.1% of cells in the genetically modified cell population is subjected to gene editing at a certain site, an actual gene modification ratio of the site cannot be accurately measured by an existing deep sequencing detection method. In other words, due to an error existing in the existing deep sequencing process, even if there are no genetically modified cells, a ratio of the genetically modified cells obtained by using the existing deep sequencing detection method is also a numerical value of 0-0.1%. The existing deep sequencing detection method cannot distinguish a condition that no gene modification occurs or the gene modification ratio is equal to or less than about 0.1%. Therefore, if a detection value of the gene editing efficiency is lower than about 0.1%, it is generally considered as background of the detection method. For some special sites, the detection background value is higher at a site having a higher basic mutation value. Therefore, whether the off-target effect exists is generally described based on existence of significant difference. For example, compared with control cells that are not subjected to gene editing modification, sequence variation is less than 2 times that of control cells at the off-target tagged site (through detection, a ratio of a proportion of the gene sequence variation of the cells in the genetically modified cell population occurring at the off-target tagged site to a proportion of the gene sequence variation of the control cells that are not subjected to gene editing modification occurring at the off-target tagged site is less than 2); and it may be considered that, no gene editing occurs at the site. Correspondingly, for the sequence variation of more than 2 times (including 2 times), it may be determined that gene editing occurs at the site, referring to Maeder, Morgan L., et al. "Development of a gene-editing approach to restore vision loss in Leber congenital amaurosis type 10." Nature medicine 25.2 (2019): 229.

In the gene editing field, the gene editing efficiency is generally defined as a ratio of indel (insertion/deletion) occurring in all enzyme action sites. For example, by conducting deep sequencing for 200000 times for a certain specific site in 20000 sets of chromosomes of 10000 cells, a frequency of occurrence of the indel is 100 times, and then the gene editing efficiency is 100/200000=0.05%.

In some embodiments of the above method in the present application, the genetically modified cells are CD34 positive hematopoietic stem cells/progenitor cells; and BCL11A genes of the cells are subjected to gene editing modification by the CRISPR/Cas system. In some embodiments of the present invention, a BCL11A enhancer is disrupted by a "CRISPR/Cas" gene editing method (a nucleotide sequence that affects or enhances the expression or function of the BCL11A). The BCL11A enhancer refers to Bauer et al., P253-257, 2013, Volume 342, Science. An instance of the BCL11A enhancer is a nucleotide sequence between exon 2 and exon 3 of the BCL11A gene (e.g., nucleic acids located at or corresponding to sites at locations such as +55:Chr2:60497676-60498941, +58:Chr2:60494251-60495546 and +62:Chr2:60490409-60491734 recorded in hg38). One instance of the BCL11A enhancer is an region +62 of a nucleotide sequence between the exon 2 and exon 3 of the BCL11A gene. One instance of the BCL11A enhancer is an region +58 of a nucleotide sequence between the exon 2 and exon 3 of the BCL11A gene (a 150 bp sequence of a site 58K of the BCL11A gene: ctgccagtcctcttctaccccacccacgcccccaccctaatcagaggcca aacccttcctggagcctgtgataaaagcaactgttagcttgcactagactagcttcaaagttgtattgaccctggtgtgttatgtctaagagtagatg cc) (SEQ ID NO: 2). In some embodiments, the BCL11A enhancer is an region +55 of a nucleotide sequence between the exon 2 and exon 3 of the BCL11A gene.

In some embodiments of the above method, the function of the BCL11A is decreased by modifying the BCL11A gene in an region from the site 60495197 to the site 60495346 of the human Chromosome 2. In some embodiments of the above method, the CD34 positive HSPC cells are modified by the CRISPR/Cas technology so as to decrease the function of the BCL11A. In some embodiments of the above method, a BCL11A genomic region from the site 60495219 to the site 60495336 on Chromosome 2 of the hematopoietic stem cells is disrupted by the gene editing technology so as to decrease the BCL11A function. In some embodiments of the above method, a target nucleotide sequence of the BCL11A genome is complementary to any sequence selected from SEQ ID No.3-SEQ ID No.25.

In some embodiments of the above method, sgRNA of any sequence selected from SEQ ID No.3-SEQ ID No.25 is introduced into the CD34 positive hematopoietic stem cells/progenitor cells to edit the BCL11A gene so as to decrease the BCL11A function. In some embodiments of the above method, the sgRNA is modified through 2'-O-methyl analogs and/or 3' sulfo between the nucleotides. In some embodiments of the above method, the chemical modification is 2'-O-methyl analog modification of the first one, two and/or three bases of the 5' end of the sgRNA and/or the last base of the 3' end. In some embodiments of the above method, the sgRNA and Cas9 encoding nucleotides are introduced into the CD34 positive hematopoietic stem cells/progenitor cells together. In some embodiments of the above method, the sgRNA and the Cas9 encoding nucleotides are introduced into the hematopoietic stem cells by electroporation. In some embodiments of the above method, the electroporation conditions are 200-600 V, 0.5-2 ms.

As used in the present invention, the "BCL11A" is a transcription factor, is discovered in mice first, serves as a binding site of retrovirus and is named as Evi9. Later, the gene is discovered in human genome and is located at a short-arm site 2p13 of the Chromosome 2.

Decrease of the BCL11A function through modification is as follows: the BCL11A gene is disrupted through gene modification, i.e., through gene editing by the CRISPR/Cas system and method.

In some embodiments of the above method, a gene modification target site is a site in the BCL11A gene complementary to any sequence selected from SEQ ID No.3-SEQ ID No.25.

Table 1 lists genome sequence locations on human Chromosome 2 targeted by the sgRNA including any sequence as shown in SEQ ID No.3-SEQ ID No.25, and Cas9 cutting sites initiated by each sgRNA.

**Table 1:**

| Name | Locations on human genome sequences | Cutting sites |
|---|---|---|
| Enhancer-7 of BCL11A | chr2: 60495203-60495222 | chr2:60495219 |
| Enhancer-8 of BCL11A | chr2: 60495208-60495227 | chr2:60495224 |
| Enhancer-9 of BCL11A | chr2:60495217-60495236 | chr2:60495233 |
| Enhancer-10 of BCL11A | chr2:60495218-60495237 | chr2:60495234 |
| Enhancer-11 of BCL11A | chr2:60495219-60495238 | chr2:60495235 |
| Enhancer-14 of BCL11A | chr2:60495221-60495240 | chr2:60495223 |
| Enhancer-12 of BCL11A | chr2: 60495222-60495241 | chr2:60495238 |
| Enhancer-13 of BCL11A | chr2: 60495223-60495242 | chr2:60495239 |
| Enhancer-15 of BCL11A | chr2: 60495228-60495247 | chr2:60495244 |
| Enhancer-16 of BCL11A | chr2: 60495229-60495248 | chr2:60495245 |
| Enhancer-17 of BCL11A | chr2: 60495230-60495249 | chr2:60495246 |
| Enhancer-18 of BCL11A | chr2:60495231-60495250 | chr2:60495247 |
| Enhancer-19 of BCL11A | chr2: 60495234-60495253 | chr2:60495250 |
| Enhancer-20 of BCL11A | chr2: 60495235-60495254 | chr2:60495251 |
| Enhancer-2 of BCL11A | chr2: 60495236-60495255 | chr2:60495238 |
| Enhancer-1 of BCL11A | chr2:60495247-60495266 | chr2:60495263 |
| Enhancer-6 of BCL11A | chr2: 60495252-60495271 | chr2:60495268 |
| Enhancer-5 of BCL11A | chr2: 60495253-60495272 | chr2:60495269 |
| Enhancer-4 of BCL11A | chr2:60495257-60495276 | chr2:60495273 |
| Enhancer-3 of BCL11A | chr2: 60495264-60495283 | chr2:60495280 |
| Enhancer-22 of BCL11A | chr2:60495299-60495318 | chr2:60495301 |
| Enhancer-23 of BCL11A | chr2:60495319-60495338 | chr2:60495335 |
| Enhancer-21 of BCL11A | chr2: 60495320-60495339 | chr2:60495336 |

SEQ ID NO: 3: sgRNA named as enhancer-1 of BCL11A (sometimes called enhancer-1 for short): cacaggctccaggaagggtt
SEQ ID NO: 4: sgRNA named as enhancer-2 of BCL11A (sometimes called enhancer-2 for short): atcagaggccaaacccttcc
SEQ ID NO: 5: sgRNA named as enhancer-3 of BCL11A (sometimes called enhancer-3 for short): Ctaacagttgcttttatcac
SEQ ID NO: 6: sgRNA named as enhancer-4 of BCL11A (sometimes called enhancer-4 for short): ttgcttttatcacaggctcc
SEQ ID NO: 7: sgRNA named as enhancer-5 of BCL11A (sometimes called enhancer-5 for short): ttttatcacaggctccagga
SEQ ID NO: 8: sgRNA named as enhancer-6 of BCL11A (sometimes called enhancer-6 for short): tttatcacaggctccaggaa
SEQ ID NO: 9: sgRNA named as enhancer-7 of BCL11A (sometimes called enhancer-7 for short): tgggtggggtagaagaggac
SEQ ID NO: 10: sgRNA named as enhancer-8 of BCL11A (sometimes called enhancer-8 for short): gggcgtgggtggggtagaag
SEQ ID NO: 11: sgRNA named as enhancer-9 of BCL11A (sometimes called enhancer-9 for short): ttagggtgggggcgtgggtg
SEQ ID NO: 12: sgRNA named as enhancer-10 of BCL11A (sometimes called enhancer-10 for short): attagggtgggggcgtgggt
SEQ ID NO: 13: sgRNA named as enhancer-11 of BCL11A (sometimes called enhancer-11 for short): gattagggtgggggcgtggg
SEQ ID NO: 14: sgRNA named as enhancer-12 of BCL11A (sometimes called enhancer-12 for short): tctgattagggtgggggcgt
SEQ ID NO: 15: sgRNA named as enhancer-13 of BCL11A (sometimes called enhancer-13 for short): ctctgattagggtgggggcg
SEQ ID NO: 16: sgRNA named as enhancer-14 of BCL11A (sometimes called enhancer-14 for short): cacgcccccaccctaatcag
SEQ ID NO: 17: sgRNA named as enhancer-15 of BCL11A (sometimes called enhancer-15 for short): ttggcctctgattagggtgg
SEQ ID NO: 18: sgRNA named as enhancer-16 of BCL11A (sometimes called enhancer-16 for short): tttggcctctgattagggtg
SEQ ID NO: 19: sgRNA named as enhancer-17 of BCL11A (sometimes called enhancer-17 for short): gtttggcctctgattagggt
SEQ ID NO: 20: sgRNA named as enhancer-18 of BCL11A (sometimes called enhancer-18 for short): ggtttggcctctgattaggg
SEQ ID NO: 21: sgRNA named as enhancer-19 of BCL11A (sometimes called enhancer-19 for short): aagggtttggcctctgatta
SEQ ID NO: 22: sgRNA named as enhancer-20 of BCL11A (sometimes called enhancer-20 for short): gaagggtttggcctctgatt
SEQ ID NO: 23: sgRNA named as enhancer-21 of BCL11A (sometimes called enhancer-21 for short): actcttagacataacacacc
SEQ ID NO: 24: sgRNA named as enhancer-22 of BCL11A (sometimes called enhancer-22 for short): cttcaaagttgtattgaccc
SEQ ID NO: 25: sgRNA named as enhancer-23 of BCL11A (sometimes called enhancer-23 for short): ctcttagacataacacacca

Through analysis of the cutting sites of the above 23 sgRNA, the Cas9 cutting locations initiated by the sgRNA are concentrated in a genomic region from the site 60495219 to the site 60495336 of the BCL11A gene.

Generally speaking, a guide sequence in the sgRNA refers to any polynucleotide sequence that has enough complementarity with a target polynucleotide sequence to hybridize with a target sequence and guide a CRISPR complex to be specifically bound with the target sequence. In some embodiments, when optimal alignment is achieved by using an appropriate alignment algorithm, a complementary degree between the guide sequence and the corresponding target sequence thereof is about or more than about 80%, 85%, 90%, 95%, 97.5%, 99% or more. The optimal alignment may be determined by using any appropriate algorithm used for sequence alignment; and non-restrictive instances of the algorithms include Smith-Waterman algorithm, Needleman-Wimsch algorithm, algorithm based on Burrows-Wheeler Transform (such as Burrows Wheeler Aligner), ClustalW, Clustai X, BLAT, Novoalign (Novocraft Technologies, ELAND ((Illumina, San Diego, CA), SOAP (available on soap.genomics.org.cn) and Maq (available on maq.sourceforge.net). In some embodiments, the length of the guide sequence is about 19-21, such as 19 bp, 20 bp, and 21 bp.

In one aspect, the present application relates to a method for determining the suitability of a cell therapy for an individual. Specifically, the present invention provides the method for determining the suitability of the cell therapy for the individual. The method includes a step of administering a genetically modified cell population from an individual to the individual, wherein the genetically modified cells are modified at a target site through gene editing. The method includes the following steps:
a) determining the occurrence of gene editing of each site in multiple off-target tagged sites in the genetically modified cells or offspring thereof; and
b) assessing the suitability of the therapy based on the occurrence,
wherein the nonoccurrence of gene editing at any of the multiple off-target tagged sites indicates that the cell therapy is suitable for the individual.

In another aspect, the present invention further provides a method for determining unsuitability of a cell therapy for an individual. The method includes a step of administering a genetically modified cell population from an individual to the individual, wherein the genetically modified cells are modified at a target site through gene editing. The method includes the following steps:
a) determining the occurrence of gene editing of each site in multiple off-target tagged sites in the genetically modified cells or offspring thereof; and
b) assessing the suitability of the therapy based on the occurrence,
wherein the occurrence of gene editing at any of the multiple off-target tagged sites indicates that the cell therapy is unsuitable for the individual.

In some embodiments, the offspring of the genetically modified cell refers to daughter cells obtained after cell division of the genetically modified cells. In some embodiments, the offspring of the genetically modified cell refers to daughter cells differentiated from the genetically modified cells. In some embodiments, the genetically modified cells are gene modified hematopoietic stem cells. In some embodiments, the offspring of the genetically modified cell refers to differentiated offspring of the gene modified hematopoietic stem cells.

The "hematopoietic stem cells" refer to a cell population with great proliferation potential, multi-directional differentiation ability and self-renewal ability. The hematopoietic stem cells can differentiate and supplement various blood cells and further maintain properties and quantities of the stem cells through self-renewal. The hematopoietic stem cells have different differentiated degrees and proliferation abilities and have heterogeneity. Pluripotential hemopoietic stem cells are the most primitive, are first differentiated into committed pluripotential hemopoietic stem cells and can produce granuloid, red, mononuclear and megakaryon-platelet myeloid hematopoietic stem cells and produce lymphoid stem cells of B lymphocytes and T lymphocytes. The two types of stem cells maintain basic characteristics of the hematopoietic stem cells, are slightly differentiated, and are in charge of production of "marrow components" and lymphocytes. Therefore, the stem cells are called the committed pluripotential hemopoietic stem cells. Then, the committed pluripotential hemopoietic stem cells are further differentiated into hemopoietic progenitor cells. Although the cells are primitive blood cells, these cells have lost many basic characteristics of the hemopoietic stem cells, e.g., the cells have lost multi-directional differentiation abilities and can only be differentiated into primary cells or closely related secondary cells. The repeated self-renewal ability is lost; and the quantity needs to be supplemented by virtue of proliferation and differentiation of the hematopoietic stem cells. The cells have limited proliferation potential and can only be divided for several times. According to the quantity of blood cell lines differentiated by the hemopoietic progenitor cells, the hemopoietic progenitor cells are differentiated into unipotent hemopoietic progenitor cells (only differentiated into a blood cell line) and oligomeric hemopoietic progenitor cells (may be differentiated into 2-3 blood cell lines). In the present application, the term "hematopoietic stem cell/progenitor cell" and the term "hematopoietic stem cell" may be interchangeable, include the pluripotential hemopoietic stem cells, the committed pluripotential hemopoietic stem cells and the hemopoietic progenitor cells, and are generic terms of the hemopoietic stem cells having different heterogeneity.

In some embodiments, the genetically modified cells in the above method are gene modified CD34 positive hematopoietic stem cells/progenitor cells. The CD34 positive hematopoietic stem cells/progenitor cells may be detected and counted by using flow cytometry and a fluorescently-labeled anti-CD34 antibody.

In specific embodiments, the CD34 positive hematopoietic stem cells/progenitor cells are isolated or obtained from an organism (individual) containing hematopoietic cells. The "isolation" is to take the cells out of an original environment. For example, when the cells are isolated from some or all components accompanying with the cells generally in a natural state, the cells are isolated.

The hematopoietic stem cells/progenitor cells may be obtained or isolated from unfractionated or fractionated marrow of an adult, and include cells in thighbone, hip bone, ribs, sternum and other skeletons. The hematopoietic stem cells/progenitor cells may be directly obtained or isolated from the hip bone by utilizing a needle or a syringe, or obtained from blood, and are generally obtained from the blood after pretreated with a hematopoietic stem cell mobilization agent such as G-CSF (granulocyte colony-stimulating factor). Other sources of the hematopoietic stem cells and progenitor cells include umbilical cord blood, placental blood and mobilized peripheral blood of the individual.

After isolated from the individual (such as from the marrow or peripheral blood), the obtained cell population may be further purified to obtain the CD34 positive hematopoietic stem cells/progenitor cells. For example, mature lineage committed cells in the isolated cell population may be removed by immunization, e.g., a solid matrix is labeled by using antibodies of a group of "lineage" antigens (such as CD2, CD3, CD11b, CD14, CD15, CD16, CD19, CD56, CD123 and CD235a); and then the primitive hematopoietic stem cells and progenitor cells are isolated by using an antibody binding with a CD34 positive antigen. A kit used for purifying the hematopoietic stem cells and progenitor cells from multiple cell sources is commercially available.

In some specific embodiments of the present application, the genetically modified cells are the isolated CD34 positive hematopoietic stem cells/progenitor cells, i.e. the cells in which the BCL11A gene are disrupted through gene editing methods such as a CRISPR technology.

Cell "differentiation" refers to a process of enabling cells of the same source to gradually produce cell population of different morphological structures and functional characteristics.

"Differentiation" from the hematopoietic stem cells to the erythrocytes includes a hematopoietic stem cell stage, a erythroid progenitor cell stage, a proliferation and differentiation stage of erythroid precursor cells (from primary erythrocytes to late erythrocytes), a proliferation and maturation process of reticulocyte, and a stage of releasing the reticulocyte to the peripheral blood to be maturated into the erythrocytes. The hematopoietic stem cell stage is as follows: as it is known that, the hematopoietic stem cells mainly exist in hemopoietic tissues such as marrow, spleen and liver. Some hematopoietic stem cells circulate in the peripheral blood. The erythroid progenitor cell stage is as follows: at the erythroid progenitor cell stage, the cells are cell population located between the hematopoietic stem cells and the erythroid precursor cells. The hematopoietic stem cells are differentiated into the erythroid progenitor cells under the effect of a hematopoietic inductive microenvironment. The hematopoietic inductive microenvironment includes a microvascular system, a nervous system, a mesenchymal part and the like and has special effects and influences on the differentiation of the hematopoietic stem cells through a humoral factor and a cytokine. The erythroid precursor cell stage includes various stages of primitive erythrocytes, basophilic erythroblast, polychromatic erythroblast, orthochromatic normoblast and reticulocyte for producing mature erythrocyte.

The cell maturation process is a process of increasing hemoglobin and decreasing cell nucleus activity. With maturation of the cells, hemoglobin content in nucleated erythrocytes is continuously increased, while RNA content is continuously decreased. Due to increase of the hemoglobin in the erythrocytes, the nucleus activity is lost, and the DNA or RNA is not synthesized any more. Experiments had proved the reason is that the hemoglobin enters the nucleus via pores among nuclear membranes and acts on nucleohistones, chromosome inactivation is initiated so as to promote nuclear condensation. The orthochromatic normoblast has lost continuous division ability, causes nuclear pyknosis and escape. Then, the orthochromatic normoblast is subjected to phagocytosis by mononuclear macrophage or is divided or dissolved in spleen so as to form enucleated reticulocyte. The hemoglobin is not synthesized at the mature erythrocyte stage. According to the theory that the cell nucleus activity is lost due to increase of a hemoglobin concentration in the cells, the division times in the erythrocyte maturation process and the final size of the cells have certain correlation to the hemoglobin synthesis rate. With maturation of the cells, diameters of erythroid cells are gradually decreased, and cell volumes are also gradually decreased, which is because some organelles (such as mitochondria, Golgi's organs and polyribosome) used for synthesizing the hemoglobin, matrix protein and various enzymes in the cells are gradually decreased and also gradually degenerated to disappear.

In some embodiments of the present invention, the offspring of the genetically modified cell is differentiated offspring of the hematopoietic stem cells, such as the nucleated erythrocytes at various differentiation stages in the peripheral blood, including basophilic normoblast, polychromatic erythroblast or orthochromatic normoblast.

In one aspect, the present invention relates to a method for treating an individual. Specifically, the present invention provides a cell therapy. The cell therapy includes: 1) a step of determining suitability of the cell therapy for an individual; and 2) a step of administering a genetically modified cell population from an individual to the individual suitable for the cell therapy, wherein the genetically modified cells are modified at a target site through gene editing. The steps of determining the suitability of the cell therapy for the individual includes:
a) determining the occurrence of gene editing of each site in multiple off-target tagged sites in the genetically modified cells or offspring thereof; and
b) assessing the suitability of the therapy based on the occurrence,
wherein the nonoccurrence of gene editing at any of the multiple off-target tagged sites indicates that the cell therapy is suitable for the individual.

In some embodiments of the above method, the gene editing modified cell population is not proliferated ex vivo or in vitro before administered to the individual. In specific embodiments, the gene editing modified cells may be washed to remove treatment reagents and then administered to the patients without being proliferated ex vivo. In some embodiments, the gene editing modified cells are administered to the patients before any obvious in-vitro cell division or before the time needed by any obvious in-vitro cell division. In some embodiments, after the modification and before administered to the individual, the gene editing modified cells are cultured for one or more days . In some embodiments, the modified cells are administered to the patients within 2, 4, 6, 12, 24 and 48 hours after modification.

In some embodiments, before the modified cells are administered to the individual, the modified cells are stored under freezing conditions for at least 24 hours. In some embodiments, before stored under the freezing conditions, the modified cells are cultured for one or more days. The modified cells may be cultured in a culture medium added with cytokines that maintain cell viability on the basis of a serum-free basal culture medium for one or more days (such as 1-3 days).

In some embodiments, the modified cells are administered via intravenous injection, e.g., the modified cells are administered once a week,once every two weeks, once every three weeks and once every month or two months. A number of the administered cells is about 2×10⁵-2×10⁷ cells per kilogram of body weight.

The term "therapy" used in the present invention refers to achieved pharmacological and/or physiological effects, including but not limited to improvement or elimination of disease symptoms. The effect may be preventative and shows that the diseases or symptoms thereof are completely or partially prevented; and/or the effect may be therapeutic and shows that the symptoms are improved or eliminated, or provides partial or complete healing of the diseases and/or adverse effects attributive to the diseases. As used in the present invention, the "therapy" includes any therapy to diseases of mammals, particularly humans, including: (a) preventing onset of the individual; (b) inhibiting the diseases, i.e., preventing development of the diseases; (c) alleviating the diseases, such as disease elimination, i.e., complete or partial elimination of the disease symptoms; and (d) enabling the individual to restore to a state before the diseases, such as reconstitution of a hemopoietic system. In the present application, the "therapy" does not necessarily mean complete elimination or healing of the diseases or disease states or related symptoms thereof, and covers any minimum improvement or alleviation of any or more measurable representation of the diseases or disease states. In the above specific method, the therapy includes improvement of hematopoietic reconstitution or survival of the individual.

In one aspect, the present invention provides a method for diagnosing whether off-target occurs accompanying with an individual gene editing cell therapy. The method includes: a) administering a genetically modified cell population from an individual to the individual, wherein the genetically modified cells are modified at a target site through gene editing; and
b) determining the occurrence of gene editing of each site in multiple off-target tagged sites in the genetically modified cells or offspring thereof,
wherein the nonoccurrence of gene editing at any of the multiple off-target tagged sites indicates that the cell therapy has no off-target.

In one aspect, the present invention provides a method for monitoring an effect of a gene editing cell therapy. The method includes: a) administering a genetically modified cell population from an individual to the individual, wherein the genetically modified cells are modified at a target site through gene editing; and
b) determining the occurrence of gene editing of each site in multiple off-target tagged sites in the genetically modified cells or offspring thereof,
wherein the nonoccurrence of gene editing at any of the multiple off-target tagged sites indicates that the cell therapy has the treatment effect on the individual.

In one aspect, the present invention provides a method for monitoring quality of a genetically modified cell product from a certain individual. The method includes: a) determining the occurrence of gene editing of each site in multiple off-target tagged sites in the genetically modified cells,
wherein the nonoccurrence of gene editing at any of the multiple off-target tagged sites indicates that the cell product is suitable for the individual for conducting cell therapy.

In some embodiments, the method includes administration of the genetically modified cell product to the individual.

In one aspect, the present invention provides a method for judging whether an intervention therapy needs to be administered to an individual of the gene editing cell therapy. The method includes: a) administering a genetically modified cell population from an individual to the individual, wherein the genetically modified cells are modified at a target site through gene editing; and
b) determining the occurrence of gene editing of each site in multiple off-target tagged sites in the genetically modified cells or offspring thereof,
wherein the occurrence of gene editing at any of the multiple off-target tagged sites indicates that the intervention therapy needs to be administered to the individual of the gene editing cell therapy.

In some embodiments of the above method, the method includes conducting the determination step on the genetically modified cell before administering the genetically modified cell to the individual. In some embodiments of the above method, the method further includes administration of the genetically modified cell to the individual. In some embodiments of the above method, the method includes conducting the determination step on the offspring of the genetically modified cell after administration of the genetically modified cells. In some embodiments of the above method, the determination step is conducted within about at least one month after the administration of the genetically modified cell, such as at least 30 days, 40 days, 50 days and 60 days. In some embodiments of the above method, the method further includes conducting the determination and assessment steps once or repeatedly, such as 2 times, three times, four times, five times or more times. In some embodiments of the above method, the determination and assessment steps are repeated at a frequency of about once every month to about once every year, e.g., the steps are repeated at a frequency of once about every month, about every two months, about every three months, about every four months, about every five months, about every six months, about every seven months, about every eight months, about every nine months, about every ten months, about every eleven months and about every twelve months.

In some embodiments of the above method, the method intervention further includes treatment of the individual with the intervention therapy after the assessment step. In some embodiments of the above method, the intervention therapy is removal of the administered genetically modified cell population, including myeloablative and/or lymphcyte dissection therapy. In some embodiments of the above method, the intervention therapy includes chemotherapy, monoclonal antibody therapy or total body irradiation. In some embodiments of the above method, the chemotherapy includes administration of one or more chemotherapeutic agents selected from the following group to the individual: Busulfan, cyclophosphamide and fludarabine. In some embodiments of the above method, the intervention therapy includes administration of a second genetically modified cell population from the individual.

Sites at which the off-target occurs most possibly are predicted by the method in the present application, such as 10-50, 10-45, 10-40, 10-35, 10-30, 10-25, 10-20 and 10-15 off-target tagged sites; and then occurrence of the off-target reaction is monitored. If the off-target reaction occurs, whether a ratio of the edited sites is changed is determined. If it is clear that an off-target phenomenon occurs at a certain site, but the edited ratio of the site is constant or decreased, it is indicated that the off-target effect actually occurs at the site, while there is no risk of cell proliferation (canceration). The intervention therapy may not be adopted under this situation. If it is clear that the off-target effect occurs at a site, and with passage of time (through continuous detection), the edited ratio of the site is increasing, it is indicated that the off-target has the risk of cell proliferation (canceration). Then, the intervention therapy for the canceration shall be considered, e.g., a chemotherapeutic agent for treating the cancer is administered. Various chemotherapeutic agents for cancer therapy are known in the art.

The present invention further relates to a kit for assessing off-target editing of a genetically modified cell population or offspring thereof. The genetically modified cells are modified at the target sites through gene editing . The kit comprises: 1) one or more components of a gene editing system, used for producing the genetically modified cell; and 2) multiple primer sets used for amplifying nucleic acids comprising the multiple off-target tagged sites.

In some embodiments of the above method and kit, the gene editing method or system is selected from ZFN, TALEN, CRISPR or any other gene editing method or system. In some embodiments, the gene editing method or system is a CRISPR gene editing method or system. In some embodiments, the target sites are BCL11A gene loci in CD34 positive hematopoietic stem cells/progenitor cells. In some embodiments of the above method and kit, the target sites are nucleotide sites sequence between exon 2 and exon 3 of the BCL11A enhancer gene (e.g., located at or corresponding to sites at locations such as +55:Chr2:60497676-60498941, +58:Chr2:60494251-60495546 and +62:Chr2:60490409-60491734 recorded in hg38). The target sites are located at an region +62 of a nucleotide sequence between the exon 2 and exon 3 of the BCL11A gene, an region +58 of a nucleotide sequence between the exon 2 and exon 3 of the BCL11A gene, or an region +55 of a nucleotide sequence between the exon 2 and exon 3 of the BCL11A gene, and a nucleotide are shown as the following nucleotide sequences: ctgccagtcctcttctaccccacccacgcccccaccctaatcagaggcca aacccttcctggagcctgtgataaaagcaactgttagcttgcactagactagcttcaaagttgtattgaccctggtgtgttatgtctaagagtagatg cc (SEQ ID NO: 2). In some embodiments of the above kit, the kit comprises sgRNA targeting the above target sites, or includes a nucleotide sequence of the sgRNA, or a nucleotide sequence that encodes the sgRNA.

In some embodiments, the kit comprises sgRNA targeting the sequence shown as SEQ ID NO: 2, or includes a nucleotide sequence of the sgRNA, or a nucleotide sequence that encodes the sgRNA. In some embodiments, the kit comprises sgRNA targeting the BCL11A gene in an region from the site 60495197 to the site 60495346 of human Chromosome 2, or includes a nucleotide sequence of the sgRNA, or a nucleotide sequence that encodes the sgRNA. In some embodiments, the kit comprises sgRNA for a BCL11A genomic region from the site 60495219 to the site 60495336 of Chromosome 2 in human hematopoietic stem cells, or includes a nucleotide sequence of the sgRNA, or a nucleotide sequence that encodes the sgRNA. The nucleotide sequence of the sgRNA is selected from any or more sequences in SEQ ID No.3-SEQ ID No.25. In some embodiments, the kit further includes a Cas protein (such as a Cas9 protein), or an expression vector that encodes the Cas protein (such as the Cas9 protein), or a nucleotide sequence (such as an mRNA sequence) that encodes or expresses the Cas protein (such as the Cas9 protein). In some embodiments of the above kit, the kit comprises a Cas9 nucleotide sequence shown as follows:

gacaagaagtacagcatcggcctggacatcggcaccaactctgtgggctgggccgtgatcaccgacgagtacaaggtgcccagc
aagaaattcaaggtgctgggcaacaccgaccggcacagcatcaagaagaacctgatcggagccctgctgttcgacagcggcgaaacagccg
aggccacccggctgaagagaaccgccagaagaagatacaccagacggaagaaccggatctgctatctgcaagagatcttcagcaacgagat
ggccaaggtggacgacagcttcttccacagactggaagagtccttcctggtggaagaggataagaagcacgagcggcaccccatcttcggca
acatcgtggacgaggtggcctaccacgagaagtaccccaccatctaccacctgagaaagaaactggtggacagcaccgacaaggccgacct
gcggctgatctatctggccctggcccacatgatcaagttccggggccacttcctgatcgagggcgacctgaaccccgacaacagcgacgtgga
caagctgttcatccagctggtgcagacctacaaccagctgttcgaggaaaaccccatcaacgccagcggcgtggacgccaaggccatcctgtc
tgccagactgagcaagagcagacggctggaaaatctgatcgcccagctgcccggcgagaagaagaatggcctgttcggcaacctgattgccc
tgagcctgggcctgacccccaacttcaagagcaacttcgacctggccgaggatgccaaactgcagctgagcaaggacacctacgacgacga
cctggacaacctgctggcccagatcggcgaccagtacgccgacctgtttctggccgccaagaacctgtccgacgccatcctgctgagcgacat
cctgagagtgaacaccgagatcaccaaggcccccctgagcgcctctatgatcaagagatacgacgagcaccaccaggacctgaccctgctga
aagctctcgtgcggcagcagctgcctgagaagtacaaagagattttcttcgaccagagcaagaacggctacgccggctacattgacggcgga
gccagccaggaagagttctacaagttcatcaagcccatcctggaaaagatggacggcaccgaggaactgctcgtgaagctgaacagagagg
acctgctgcggaagcagcggaccttcgacaacggcagcatcccccaccagatccacctgggagagctgcacgccattctgcggcggcagga
agatttttacccattcctgaaggacaaccgggaaaagatcgagaagatcctgaccttccgcatcccctactacgtgggccctctggccaggga
aacagcagattcgcctggatgaccagaaagagcgaggaaaccatcaccccctggaacttcgaggaagtggtggacaagggcgcttccgccc
agagcttcatcgagcggatgaccaacttcgataagaacctgcccaacgagaaggtgctgcccaagcacagcctgctgtacgagtacttcaccg
tgtataacgagctgaccaaagtgaaatacgtgaccgagggaatgagaaagcccgccttcctgagcggcgagcagaaaaaggccatcgtgga
cctgctgttcaagaccaaccggaaagtgaccgtgaagcagctgaaagaggactacttcaagaaaatcgagtgcttcgactccgtggaaatctcc
ggcgtggaagatcggttcaacgcctccctgggcacataccacgatctgctgaaaattatcaaggacaaggacttcctggacaatgaggaaaac
gaggacattctggaagatatcgtgctgaccctgacactgtttgaggacagagagatgatcgaggaacggctgaaaacctatgcccacctgttcg
acgacaaagtgatgaagcagctgaagcggcggagatacaccggctggggcaggctgagccggaagctgatcaacggcatccgggacaag

cagtccggcaagacaatcctggatttcctgaagtccgacggcttcgccaacagaaacttcatgcagctgatccacgacgacagcctgacctttaa
agaggacatccagaaagcccaggtgtccggccagggcgatagcctgcacgagcacattgccaatctggccggcagccccgccattaagaag
ggcatcctgcagacagtgaaggtggtggacgagctcgtgaaagtgatgggccggcacaagcccgagaacatcgtgatcgaaatggccagag
agaaccagaccacccagaagggacagaagaacagccgcgagagaatgaagcggatcgaagagggcatcaaagagctgggcagccagat
cctgaaagaacaccccgtggaaaacacccagctgcagaacgagaagctgtacctgtactacctgcagaatggcgggatatgtacgtggacc
aggaactggacatcaaccggctgtccgactacgatgtggaccatatcgtgcctcagagctttctgaaggacgactccatcgacaacaaggtgct
gaccagaagcgacaagaaccggggcaagagcgacaacgtgccctccgaagaggtcgtgaagaagatgaagaactactggcggcagctgct
gaacgccaagctgattacccagagaaagttcgacaatctgaccaaggccgagagaggcggcctgagcgaactggataaggccggcttcatc
aagagacagctggtggaaacccggcagatcacaaagcacgtggcacagatcctggactcccggatgaacactaagtacgacgagaatgaca
agctgatccgggaagtgaaagtgatcaccctgaagtccaagctggtgtccgatttccggaaggatttccagttttacaaagtgcgcgagatcaac
aactaccaccacgcccacgacgcctacctgaacgccgtcgtgggaaccgccctgatcaaaaagtaccctaagctggaaagcgagttcgtgtac
ggcgactacaaggtgtacgacgtgcggaagatgatcgccaagagcgagcaggaaatcggcaaggctaccgccaagtacttcttctacagcaa
catcatgaactttttcaagaccgagattaccctggccaacggcgagatccggaagcggcctctgatcgagacaaacggcgaaaccggggaga
tcgtgtgggataagggccgggattttgccaccgtgcggaaagtgctgagcatgccccaagtgaatatcgtgaaaaagaccgaggtgcagaca
ggcggcttcagcaaagagtctatcctgcccaagaggaacagcgataagctgatcgccagaaagaaggactgggaccctaagaagtacggcg
gcttcgacagccccaccgtggcctattctgtgctggtggtggccaaagtggaaaagggcaagtccaagaaactgaagagtgtgaaagagctgc
tggggatcaccatcatggaaagaagcagcttcgagaagaatcccatcgactttctggaagccaagggctacaaagaagtgaaaaaggacctg
atcatcaagctgcctaagtactccctgttcgagctggaaaacggccggaagagaatgctggcctctgccggcgaactgcagaagggaaacga
actggccctgccctccaaatatgtgaacttcctgtacctggccagccactatgagaagctgaagggctcccccgaggataatgagcagaaaca
gctgtttgtggaacagcacaagcactacctggacgagatcatcgagcagatcagcgagttctccaagagagtgatcctggccgacgctaatctg
gacaaagtgctgtccgcctacaacaagcaccgggataagcccatcagagagcaggccgagaatatcatccacctgtttaccctgaccaatctg
ggagcccctgccgccttcaagtactttgacaccaccatcgaccggaagaggtacaccagcaccaaagaggtgctggacgccaccctgatcca
ccagagcatcaccggcctgtacgagacacggatcgacctgtctcagctgggaggcgac (SEQ ID No.1).

In some embodiments of the above method, sgRNA of any sequence selected from SEQ ID No.3-SEQ ID No.25 is introduced into the CD34 positive hematopoietic stem cells/progenitor cells to edit the BCL11A gene so as to eliminate or decrease the BCL11A function. In some embodiments of the above kit, the sgRNA is modified through 2'-O-methyl analogs and/or 3' sulfo between the nucleotides. In some embodiments of the above kit, the chemical modification is 2'-O-methyl analog modification of the first one, two and/or three bases of the 5' end of the sgRNA and/or the last base of the 3' end. In some embodiments of the above kit, the sgRNA and Cas9 encoding nucleotide are introduced into the CD34 positive hematopoietic stem cells/progenitor cells together.

In some embodiments of the above kit, the kit comprises multiple primer sets used for amplifying nucleic acids comprising the multiple off-target tagged sites. In some embodiments of the above kit, the primer sets are selected from Table 3. The sequences are numbered as SEQ ID NOs:26-117.

In some embodiments of the above method and kit, the off-target tagged sites are one or more selected from Table 2.

The present invention will be further described below in combination with specific embodiments. It should be understood that, these embodiments are merely used for describing the present invention, rather than limiting the scope of the present invention. Unmarked detailed conditions in experimental methods in the embodiments below generally refer to conditions in routine conditions or conditions suggested by manufacturers. Unless otherwise specified, experimental materials and reagents in the embodiments below are commercially available.

The present invention relates to the embodiments as follows:
1. A method for determining suitability of a cell therapy for an individual includes a step of administering a genetically modified cell population from an individual to the individual, wherein the genetically modified cells are modified at a target site through gene editing,
   the method includes the following steps:
      a) determining the occurrence of gene editing of each site in multiple off-target tagged sites in the genetically modified cells or offspring thereof; and
      b) assessing the suitability of the therapy based on the occurrence of gene editing,
   wherein the nonoccurrence of gene editing at any of the multiple off-target tagged sites indicates that the cell therapy is suitable for the individual.
2. The method according to the embodiment 1, wherein the determination step is conducted on the genetically modified cell before administering the genetically modified cells.
3. The method according to the embodiment 2, wherein the method further includes administration of the genetically modified cell to the individual.
4. The method according to the embodiment 1, wherein the determination step is conducted on the offspring of the genetically modified cell after administering the genetically modified cells.
5. The method according to the embodiment 4, wherein the determination step is conducted within at least about one month after administering the genetically modified cell, e.g., the determination step is conducted within 1 month, 1.5 months, 2 months and 2.5 months after administering the genetically modified cells.
6. The method according to the embodiment 4 or 5, further including repeating the determination and assessment steps once or more times.
7. The method according to the embodiment 6, wherein the determination and assessment steps are repeated at a frequency of about once every month to about once every year, e.g., the steps are repeated at a frequency of about every month, about every two months, about every three months, about every four months, about every five months, about every six months, about every seven months, about every eight months, about every nine months, about every ten months, about every eleven months and about every twelve months.
8. The method according to the embodiment 7, wherein if sequence variation of the off-target tagged sites is less than 0.1%, or the sequence variation of the off-target tagged sites is less than 2 times compared with that of control cells that are not subjected to gene editing modification, the determination and assessment steps are repeated at a frequency of every 3-12 months (e.g., repeated at a frequency of about every three months, about every four months, about every five months, about every six months, about every seven months, about every eight months, about every nine months, about every ten months, about every eleven months and about every twelve months); and if the sequence variation of the off-target tagged sites is more than 0.1%, 0.2%, 0.3%, 0.4%, 0.5%, 0.6%, 0.7%, 0.8%, 0.9% or 1%, or the sequence variation of the off-target tagged sites is more than 2 times, 3 times, 4 times, 5 times, 6 times, 7 times, 8 times, 9 times or 10 times compared with that of control cells that are not subjected to gene editing modification, the repetition frequency of the determination and assessment steps is increased with increase of the sequence variation frequency (e.g., the frequency is increased from a frequency of once every three months to a frequency of once every two months or once a month, or once every half month), so as to closely monitor the gene editing frequency of the off-target tagged sites and canceration risk; the higher the gene editing frequency of the off-target tagged sites is, the higher the canceration risk is; in some embodiments, when the gene editing frequency of the off-target tagged sites is monitored, occurrence of canceration is monitored in combination with other clinical examination methods, and then intervention therapy is conducted in time.
9. The method according to any of the embodiments 5-8, wherein the method further includes treatment of the individual by the intervention therapy after the assessment step, such as myeloablative and/or lymphcyte dissection therapy, chemotherapy (e.g., one or more chemotherapeutic agents administered to the individual are selected from the following group: Busulfan, cyclophosphamide and fludarabine), monoclonal antibody therapy or total body irradiation.
10. The method according to the embodiment 9, wherein the intervention therapy is removal of the genetically modified cell population from an individual administered to the individual.
11. The method according to the embodiment 9 or 10, wherein the intervention therapy includes administration of a second genetically modified cell population from the individual (a genetically modified cell population different from the administered genetically modified cell population).
12. The method according to any of the embodiments 1-11, wherein the multiple off-target tagged sites include at least about 10 off-target tagged sites.
13. The method according to any of the embodiments 1-12, wherein sequence variation of less than 0.1% occurs at the off-target tagged sites, or compared with that of control cells that are not subjected to gene editing modification, sequence variation of less than 2 times occurs at the off-target tagged sites, which indicates that no gene editing occurs at the off-target tagged sites.
14. The method according to any of the embodiments 1-13, wherein the determination step is conducted through DNA sequencing.
15. The method according to the embodiment 14, wherein the determination step includes: 1) amplifying nucleic acids comprising the multiple off-target tagged sites by using multiple primer sets; and 2) conducting sequencing analysis on the amplified nucleic acids.
16. The method according to any of the embodiments 1-15, wherein the in-vitro test method includes any one or more of the followings: BLESS, GUIDE-seq, HIGTS, Circle-seq, SITE-seq and Digenome-seq.
17. The method according to any of the embodiments 1-16, wherein the in-vitro test method is conducted under a saturation condition.
18. The method according to any of the embodiments 1-17, wherein the saturation condition allows effective cutting in at least more than 90% of the target sites.
19. The method according to the embodiment 18, wherein the saturation condition allows effective cutting in 100% of the target sites.
20. The method according to any of the embodiments 1-19, wherein the genetically modified cells are modified by the CRISPR/Cas system.
21. The method according to any of the embodiments 1-20, wherein the target sites are located at BCL11A gene loci.
22. The method according to the embodiment 21, wherein the off-target tagged sites refer to one or more of the sites as shown in Table 2.
23. A method for assessing off-target gene editing in a genetically modified cell population or offspring thereof, wherein the genetically modified cells are modified at the target sites through gene editing; the method includes determination of occurrence of gene editing of each site in the multiple off-target tagged sites; the multiple off-target tagged sites are obtained by manners as follows: 1) identifying first multiple off-target tagged sites based on their sequence similarity with the target sites; and/or 2) identifying second multiple off-target tagged sites by using an in-vitro test method; sequence variation of more than 0.1% occurs at the off-target tagged sites, or compared with that of control cells that are not subjected to gene editing modification, sequence variation of more than 2 times occurs at the off-target tagged sites, which indicates that off-target gene editing occurs in the genetically modified cell population or offspring thereof.
24. The method according to the embodiment 23, wherein the determination step is conducted through DNA sequencing.
25. The method according to the embodiment 24, wherein the determination step includes: 1) amplifying nucleic acids comprising the multiple off-target tagged sites by using multiple primer sets; and 2) conducting sequencing analysis on the amplified nucleic acids.
26. A method for obtaining multiple off-target tagged sites in a genetically modified cell population, wherein the genetically modified cells are modified at the target sites through gene editing; the method for obtaining the off-target tagged sites includes: 1) identifying first multiple off-target tagged sites based on their sequence similarity with the target sites; and/or 2) identifying second multiple off-target tagged sites by using an in-vitro test method.
27. The method according to any of the embodiments 23-26, wherein the in-vitro test method includes any one or more of the followings: BLESS, GUIDE-seq, HIGTS, Circle-seq, SITE-seq and Digenome-seq.
28. The method according to any of the embodiments 23-27, wherein the in-vitro test method is conducted under a saturation condition.
29. The method according to the embodiment 28, wherein the saturation condition allows effective cutting in more than 90% of the target sites, such as more than 91%, 92%, 93%, 94%, 95%, 96%, 97%, 98% or 99%, or 100% of the target sites.
30. The method according to any of the embodiments 23-29, wherein the genetically modified cells are modified by the CRISPR/Cas system.
31. A kit for assessing off-target editing of a genetically modified cell population or offspring thereof, wherein the genetically modified cells are modified at the target sites through gene editing ; and the kit comprises: 1) one or more components of a gene editing system, used for producing the genetically modified cell; and 2) multiple primer sets used for amplifying nucleic acids comprising the multiple off-target tagged sites.
32. The kit according to the embodiment 31, wherein the multiple off-target tagged sites are obtained by manners as follows: 1) identifying first multiple off-target tagged sites based on their sequence similarity with the target sites; and/or 2) identifying second multiple off-target tagged sites by using an in-vitro test method.
33. The kit according to any of the embodiments 31-32, wherein the gene editing system is a CRISPR/Cas9 system.
34. The kit according to any of the embodiments 31-33, further including one or more primer sets, wherein the primer sets are used for amplifying nucleic acids comprising the target sites.
35. The kit according to the embodiment 34, wherein the one or more primer sets comprise one or more of the followings: SEQ ID NOs: 26-117.

In another aspect, the present invention relates to the embodiments as follows:
1. A method for assessing off-target gene editing in a genetically modified cell population or offspring thereof, wherein the genetically modified cells are modified at the target sites through gene editing; the method includes determination of occurrence of gene editing of each site in the multiple off-target tagged sites; the multiple off-target tagged sites are obtained by manners as follows: 1) identifying first multiple off-target tagged sites based on their sequence similarity with the target sites; and/or 2) identifying second multiple off-target tagged sites by using an in-vitro test method; sequence variation of more than 0.1% occurs at the off-target tagged sites, or compared with that of control cells that are not subjected to gene editing modification, sequence variation of more than 2 times occurs at the off-target tagged sites, which indicates that off-target gene editing occurs in the genetically modified cell population or offspring thereof.
2. The method according to the embodiment 1, wherein the determination step is conducted through DNA sequencing.
3. The method according to the embodiment 2, wherein the determination step includes: 1) amplifying nucleic acids comprising the multiple off-target tagged sites by using multiple primer sets; and 2) conducting sequencing analysis on the amplified nucleic acids.
4. The method according to any of the embodiments 1-3, wherein the in-vitro test method includes any one or more of the followings: BLESS, GUIDE-seq, HIGTS, Circle-seq, SITE-seq, and Digenome-seq.
5. The method according to any of the embodiments 1-4, wherein the in-vitro test method is conducted under a saturation condition.
6. The method according to the embodiment 5, wherein the saturation condition allows effective cutting in more than 90% of the target sites.
7. The method according to any of the embodiments 1-6, wherein the genetically modified cells are modified by the CRISPR/Cas9 system.
8. The method according to any of the embodiments 1-7, wherein the target sites are located at BCL11A gene loci.
9. The method according to the embodiment 8, wherein the off-target tagged sites refer to one or more of the sites as shown in Table 2.
10. A method for obtaining multiple off-target tagged sites in a genetically modified cell population, wherein the genetically modified cells are modified at the target sites through gene editing; the method for obtaining the off-target tagged sites includes: 1) identifying first multiple off-target tagged sites based on their sequence similarity with the target sites; and/or 2) identifying second multiple off-target tagged sites by using an in-vitro test method.
11. The method according to the embodiment 10, wherein identification is conducted through DNA sequencing.
12. The method according to the embodiment 11, wherein the identification includes: 1) amplifying nucleic acids comprising the multiple off-target tagged sites by using multiple primer sets; and 2) conducting sequencing analysis on the amplified nucleic acids.
13. The method according to any of the embodiments 10-12, wherein the in-vitro test method includes any one or more of the followings: BLESS, GUIDE-seq, HIGTS, Circle-seq, SITE-seq, and Digenome-seq.
14. The method according to any of the embodiments 10-13, wherein the in-vitro test method is conducted under a saturation condition.
15. The method according to the embodiment 14, wherein the saturation condition allows effective cutting in more than 90% of the target sites.
16. The method according to any of the embodiments 10-15, wherein the genetically modified cells are modified by the CRISPR/Cas9 system.
17. A kit for assessing off-target editing of a genetically modified cell population or offspring thereof, wherein the genetically modified cells are modified at the target sites through gene editing ; and the kit comprises: 1) one or more components of a gene editing system, used for producing the genetically modified cell; and 2) multiple primer sets used for amplifying nucleic acids comprising the multiple off-target tagged sites.
18. The kit according to the embodiment 17, wherein the multiple off-target tagged sites are obtained by manners as follows: 1) identifying first multiple off-target tagged sites based on their sequence similarity with the target sites; and/or 2) identifying second multiple off-target tagged sites by using an in-vitro test method.
19. The kit according to any of the embodiments 17-18, wherein the gene editing system is a CRISPR/Cas9 system.
20. The kit according to any of the embodiments 17-19, further including one or more primer sets, wherein the primer sets are used for amplifying nucleic acids comprising the target sites.
21. The kit according to the embodiment 20, wherein the one or more primer sets comprise one or more of the followings: SEQ ID NOs: 26-117.

### Examples

### Example 1: Confirming that sgRNA targeting a BCL11A enhancer has only one erythroid-specific enhancer target site of a BCL11A gene that is completely matched on entire human genome

A CRISPR/Cas9 system precisely guides a Cas9 protein to the target location for gene modification for a specific target of the erythroid-specific enhancer of the BCL11A gene by utilizing a binding domain sequence of 20 bases on the sgRNA.

Logging in UCSC genome browser (https://genome.ucsc.edu/) through a browser; selecting BLAT (rapidly align sequences to the genome in Our tools on the right of the webpage, wherein the BLAT tool can find all domains that are completely matched with queried sequences in the human genome; determining that the selected genome is the human genome (version GRCh38/hg38); filling the binding domain sequence of the sgRNA (CTAACAGTTGCTTTTATCAC, SEQ ID NO: 5), including 20 bases, in the search box; and then clicking submit.

Returning after search on the UCSC. Only one unique result on Chromosome 2 is returned in the search result, i.e., the sgRNA has only one completely matched site on the human genome. The location is the target site of the sgRNA, i.e., the specific target for the erythroid- specific enhancer of the BCL11A gene. It is confirmed that, the sgRNA has only one completely matched site on the entire human genome, i.e., the specific target for the erythroid-specific enhancer of the BCL11A gene.

### Example 2: Bioinformatic method for predicting and analyzing potential off-target site of gene editing

Mismatch of a certain probability may exist during binding of sgRNA and genomic DNA sequences, thereby causing off-target mutation at an off-target site. Viewing from this point, the off-target site certainly has high similarity with the sgRNA targeting sequence. Therefore, in the present study, Cas-OFFinder (http://www.rgenome.net/cas-offinder/) software is selected first; a sequence similar to the target site of the sgRNA is aligned through whole genome; and then the potential off-target site is found.

Logging in Cas-OFFinder (http://www.rgenome.net/cas-offinder/) through a browser; selecting SpCas9 from Streptococcus pyogenes :5'-NGG-3' in PAM Type options on the left of the webpage; selecting Homo sapiens (GRCh38/hg38) - Human in the Target Genome option; filling in sgRNA sequence: CTAACAGTTGCTTTTATCAC, Mismatch Number: 3 on the right of the webpage;;and filling in 1 at DNA/RNA Bulge Size.

Eliminating a sequence with co-existence of 3 Mismatch and bulge after returning the result; then removing repeated items at the same site by returning location information of the site on the chromosome; and finally obtaining the potential off-target site of the sgRNA on the human genome.

### Example 3: Digenome-seq experimental method for predicting and analyzing potential off-target site of gene editing

In a genome editing process, genome DNA is cut by a Cas protein to produce double strand break (DSB); a Cas protein binding location may be identified by recognizing a genome DSB region; and an off-target effect may be further detected. A whole genome off-target effect of gene editing is detected based on this principle.

After characteristics of multiple gene editing off-target methods are known in detail, existing solutions of the gene editing therapy are compared. In the present example, Digenome-seq with the highest sensitivity is selected as another independent method to be used for searching the potential off-target site of the sgRNA.

The human genome DNA is extracted from K562 cells; primers for the BCL11A site are synthesized by Rui Biotechnology Co., Ltd.; the sgRNA is obtained from Synthego Company; and the spCas9 protein and buffer come from NEB Company (M0386).

The genome is extracted from 10⁶ K562 cells; the sgRNA targeting region is amplified with primers and then serves as positive control of cutting; during experiment, 2 parts of Cas9 cutting system are prepared with 1 µl of Cas9 protein (NEB M0386), 3 µl of Cas9 buffer, 1.5 µl of sgRNA and 14.5 µl of water; and the Cas9 cutting system is placed in a warm bath at 25°C to make Cas9 bind with the sgRNA. K562 genome is added; a reaction is carried out at 37°C for 8 hours; and the product is sent for sequencing after the reaction is ended. Data analysis is conducted in accordance with literatures (Kim D, Bae S, Park J, Kim E, Kim S, et al. 2015. Digenome-seq: genome-wide profiling of CRISPRCas9 off-target effects in human cells. Nat. Methods 12:237-43). In short, a sequencing file fq.gz is unzipped to obtain files sam; for each sam, the sam is respectively extracted according to chromosome chrl-XY so as to obtain chr.sort.bam; each corresponding chr.vcf.txt is calculated for the chr.sort.sam that corresponds to each chromosome; a vertical cutting site is searched for a file vcf of each chromosome; the extracted sequence is matched with sgRNAlib for searching the sgRNA causing cutting; and for each vertical cutting site, candidate matched sgRNA may not be unique, some impossible sgRNAs are filtered according to the PAM, and the final result is listed.

In the present study, an experimental condition under which the off-target effect most likely occurs is used and is different from a preparation condition used for performing gene editing on the hematopoietic stem cells by using the system. The off-target does not necessarily occur at the potential off-target site under different conditions. Through these extreme conditions, the site of the sgRNA at which the off-target effect most likely occurs is found.

Through Digenome-seq experimental analysis, the obtained potential off-target sites of gene editing are shown as Table 2. The method in example 2 have 3 same predicted sites with this.

**Table 2: Information of target sites and off-target tagged sites**

| Name | Control genome | Prediction method | Sequence | SEQ ID NO | Chromo some | Cutting location | Match ed score |
|---|---|---|---|---|---|---|---|
| On-target | hg38 | Bio-info | CTAACAGTTGCTTTTATCACAGG | 118 | chr2 | 60495260 | 20 |
| POT-1 | hg38 | Bio-info | CTAACAGTTGC-TTTAgCACTGG | 119 | chrl6 | 64288115 | 18 |
| POT-2 | hg38 | Bio-info | CTAcCAGTTG-TTTTATCACTGG | 120 | chr2 | 161236052 | 18 |
| POT-3 | hg38 | Bio-info | CTAA-AGTTGCTTTTAaCACAGG | 121 | chr3 | 166425455 | 18 |
| POT-4 | hg38 | Bio-info | CTAACtaTTGCTTTTATCAGCAGG | 122 | chr1 | 154757221 | 17 |
| POT-5 | hg38 | Bio-info | gTAACAGTTGCTTTTgTCACCAGG | 123 | chr2 | 52353784 | 17 |
| POT-6 | hg38 | Bio-info | CcAA-AGTTGCcTTTATCACAGG | 124 | chr1 | 119592005 | 17 |
| POT-7 | hg38 | Bio-info | CTAACAGTTtCTgTT-TCACAGG | 125 | chr1 | 180126845 | 17 |
| POT-8 | hg38 | Bio-info | CTAgaAGTTGCTTTT-TCACAGG | 126 | chr1 | 193995670 | 17 |
| POT-9 | hg38 | Bio-info | CTAAtA-TTGCTTTTtTCACAGG | 127 | chr11 | 103923360 | 17 |
| POT-10 | hg38 | Bio-info | CaAACAGTTGCTcTT-TCACTGG | 128 | chrl2 | 39267607 | 17 |
| POT-11 | hg38 | Bio-info | CTAACAGTTG-TTTTATgtCTGG | 129 | chrl2 | 74831072 | 17 |
| POT-12 | hg38 | Bio-info | gTAACAGcTGCTTTTA-CACAGG | 130 | chrl2 | 83538243 | 17 |
| POT-13 | hg38 | Bio-info | CaAACA-TcGCTTTTATCACGGG | 131 | chrl3 | 61796559 | 17 |
| POT-14 | hg38 | Bio-info | CTtACAGTTtCTTTTAT-ACAGG | 132 | chrl3 | 61848466 | 17 |
| POT-15 | hg38 | Bio-info | CTgACAGTTGC-TgTATCACTGG | 133 | chrl3 | 106090756 | 17 |
| POT-16 | hg38 | Bio-info | CTAAtAGTT-CTTTgATCACTGG | 134 | chrl4 | 21323544 | 17 |
| POT-17 | hg38 | Bio-info | CTgACAGTTGCTTTTA-CtCTGG | 135 | chrl6 | 12132950 | 17 |
| POT-18 | hg38 | Bio-info | CTAACA-TTGCTTaTATCtCAGG | 136 | chr17 | 65712444 | 17 |
| POT-19 | hg38 | Bio-info | CTAACA-aTGCTTTcATCACGGG | 137 | chr21 | 17537877 | 17 |
| POT-20 | hg38 | Bio-info | CTAgCtGTTGCTTTTAT-ACTGG | 138 | chr22 | 30099236 | 17 |
| POT-21 | hg38 | Bio-info | CTAACAGTT-CcTTTATCcCTGG | 139 | chr3 | 85803427 | 17 |
| POT-22 | hg38 | Bio-info | CTAgCAGTTGCTTTTc-CACAGG | 140 | chr3 | 139612655 | 17 |
| POT-23 | hg38 | Bio-info | CT-ACAGTTGCTaTTcTCACAGG | 141 | chr3 | 150428234 | 17 |
| POT-24 | hg38 | Bio-info | CTAACAGTTGgTTTT-TgACAGG | 142 | chr4 | 109498849 | 17 |
| POT-25 | hg38 | Bio-info | CTAACA-cTGCTTTcATCACAGG | 143 | chr4 | 146435251 | 17 |
| POT-26 | hg38 | Bio-info | CTAACAGTgGCTTTgA-CACTGG | 144 | chr5 | 10677607 | 17 |
| POT-27 | hg38 | Bio-info | CTAACAGTTcCTgTTATC-CCGG | 145 | chr5 | 52579555 | 17 |
| POT-28 | hg38 | Bio-info | tTAACAGTTG-TTTTATCAaTGG | 146 | chr6 | 16094197 | 17 |
| POT-29 | hg38 | Bio-info | gTAACAGTTGCTTgT-TCACTGG | 147 | chr6 | 118466532 | 17 |
| POT-30 | hg38 | Bio-info | CTAgaAGTTGCTTTTATC-CAGG | 148 | chr7 | 1713516 | 17 |
| POT-31 | hg38 | Bio-info | CTAAC-GTTGCTaTgATCACTGG | 149 | chr7 | 40558314 | 17 |
| POT-32 | hg38 | Bio-info | CTgtCAGTTGC-TTTATCACTGG | 150 | chr8 | 18630038 | 17 |
| POT-33 | hg38 | Bio-info | tTcACA-TTGCTTTTATCACTGG | 151 | chrX | 49982798 | 17 |
| POT-34 | hg38 | Bio-info | CcAACAGTTGCTTTTcTCtCTGG | 152 | chr11 | 63955658 | 17 |
| POT-35 | hg38 | Bio-info | CTAACAGTTaCTTTTATtcCTGG | 153 | chrl3 | 30199212 | 17 |
| POT-36 | hg38 | Merged | CTAACAaTTGgTTTTATaACAGG | 154 | chr5 | 51113378 | 17 |
| POT-37 | hg38 | Bio-info | CTAACAtTTGaTTTTAaCACTGG | 155 | chr8 | 87700771 | 17 |
| POT-38 | hg38 | Merged | CTAACAGcTGCTTTTATCctGGG | 156 | chr8 | 140143017 | 17 |
| POT-39 | hg38 | Merged | CTAACAtcTGCTTTTATCcCAGG | 157 | chrX | 101733819 | 17 |
| POT-40 | hgl9 | Digenome -seq | aTtACAGcTGCaTTTATCACAGG | 158 | chr10 | 34042158 | 16 |
| POT-41 | hgl9 | Digenome -seq | gagACAGTaGCTTTTATaACAGG | 159 | chrl8 | 20142254 | 15 |
| POT-42 | hgl9 | Digenome -seq | CaAtCAGcTGCTcTT A TaACTGG | 160 | chr4 | 122580132 | 15 |
| POT-43 | hgl9 | Digenome -seq | tctACcagTtgTTTT A TCACTGG | 161 | chr2 | 162092470 | 12 |
| POT-44 | hgl9 | Digenome -seq | agcACAGTTGgTTTTtatAaCAG | 162 | chr4 | 162312144 | 12 |
| POT-45 | hgl9 | Digenome -seq | agcAtAGTTGCTTTTtatcaCAG | 163 | chr11 | 16533856 | 11 |

Bio-info represents: obtained through bioinformatic prediction;
Digenome represents: obtained through a Digenome-seq method; and
Both represents: obtained through the both methods parallelly.
for the predicted potential off-target sites of the sgRNA, other five prediction methods are respectively shown as follows:
BLESS: to ligate double-strand broken DNA ends with biotin, then enrich it with streptavidin and perform sequencing and analyis;
GUIDE-seq: insert dsODN (double-stranded oligodeoxynucleotide) into a DNA double-strand break region for labelling DSB and perform sequencing and analysis;
HTGTS: ligate two double-strand break regions for labelling with translocation and perform sequencing and analysis;
Circle-seq: label the cutting sites in vitro and perform sequencing and analysis; and
SITE-seq: label the cutting sites in vitro and perform sequencing and analysis.

### Example 4: Construction of potential off-target recognition tag for sgRNA

The sequences obtained by the bioinformatic prediction method and the DiGenome-seq analysis method are merged; and the repeated parts are removed so as to obtain the potential off-target recognition tag of the sgRNA. According to the both off-target site analysis methods of gene editing, there are totally 45 potential off-target sites at which the off-target phenomenon most likely occurs. As shown in Table 2, a specific potential off-target recognition tag may be formed.

The Table 2 is a certain potential off-target site of the sgRNA targeting the BCL11A enhancer.

### Example 5: Detection of off-target frequency of samples for the potential off-target recognition tag

Through fragment PCR, the potential off-target recognition tag (including 45 potential off-target sites) constructed in example 4 is specifically amplified; and then information of gene editing efficiency of the target sites (located at the BCL11A enhancer) between the editing group and the untreated control group (Mock) and the potential off-target sites are compared with the deep sequencing approach, thereby determining whether the off-target effect occurs at these potential off-target sites in a process of production for treating thalassemia diseases by utilizing the sgRNA. The products are validated.

46 pairs of PCR primers (shown as Table 2) are synthesized at first, specially for the target sites and 45 potential off-target sites. The primers are synthesized by Rui Biotechnology Co., Ltd.; Taq-Hifi DNA polymerase is purchased from TransGen Biotechnology Co., Ltd. (AP131); a NEBNext Ultra II library-building kit is purchased from NEB Company (E7103); and TruSeq Dual Index Sequencing Primer Box is purchased from Illumina Company (FC-121-1003). The hematopoietic stem cells used in this experiment come from healthy donors. Untreated cells from the same donors serve as the control group.

**Table 3:**

| Site | F primer (5->3) | No. | R primer (5->3) | |
|---|---|---|---|---|
| On-target(targ et site) | CACACGGCATGGCA TACAAA | SEQ ID NO: 26 | TGAAGCTAGTCTAG TGCAAGCT | SEQ ID NO: 27 |
| POT-1 | ACCTGACTGATGGC CTAACG | SEQ ID NO: 28 | GCCATCCCCAAGCT AGGTTT | SEQ ID NO: 29 |
| POT-2 | TGTGATTCCTTCCTT CCCTTTT | SEQ ID NO: 30 | ACCTAATACATTTC TTGTCCAGTGA | SEQ ID NO: 31 |
| POT-3 | GCTATCTTGCGCCA CCATCT | SEQ ID NO: 32 | TGTACTCTGCTCTC CTTCACATC | SEQ ID NO: 33 |
| POT-4 | CATTCTCCGGGAGC AAGCAT | SEQ ID NO: 34 | GGCTGGCCTGAAGT CCATTT | SEQ ID NO: 35 |
| POT-5 | AAACACTGTAGGGG TAGAGAAGT | SEQ ID NO: 36 | AAAGCCACTGTGAC AAATACGT | SEQ ID NO: 37 |
| POT-6 | ACACCCAATCACGG TCTTGT | SEQ ID NO: 38 | ACTCTTACCCCAAA CCAATCTCTA | SEQ ID NO: 39 |
| POT-7 | CGATCCAGCTCCAC CACTTA | SEQ ID NO: 40 | CCTGGTCTCTTGGC GTTGTT | SEQ ID NO: 41 |
| POT-8 | TCTGGAAACACCCT GTTGCA | SEQ ID NO: 42 | CGCATTACACCCCT TTACTGCTA | SEQ ID NO: 43 |
| POT-9 | CCAGCTCATGGTTG CAACTG | SEQ ID NO: 44 | TGCACAGTGAAAAC CGCTCAA | SEQ ID NO: 45 |
| POT-10 | CTTCTACGCTGCCTC TTGCT | SEQ ID NO: 46 | ACTACCGATGGCAG CTATAGC | SEQ ID NO: 47 |
| POT-11 | CCATTCAGGGGCAG GTTGAT | SEQ ID NO: 48 | CCCACAAAACCAAC GGAACA | SEQ ID NO: 49 |
| POT-12 | AGCTGCTTTTACAC AGGTGAAGT | SEQ ID NO: 50 | AGTGTCTGCCTCCT ATTGCT | SEQ ID NO: 51 |
| POT-13 | CACTGGCCTGGCAA CTTTATG | SEQ ID NO: 52 | CTACTCGTCACTGG CTCTGA | SEQ ID NO: 53 |
| POT-14 | AGTCCCCTCATTCTT TCAGTTTTCA | SEQ ID NO: 54 | AACTCAAGGAAGA GATCAGTTGGA | SEQ ID NO: 55 |
| POT-15 | GCGCCCGGCTCAAC ATATTA | SEQ ID NO: 56 | CTCCTTCCTCCTCGC AGTGT | SEQ ID NO: 57 |
| POT-16 | GCATTCCAGCCTAG GCAACA | SEQ ID NO: 58 | GGAACGGTCAATTC TCAAAAGGA | SEQ ID NO: 59 |
| POT-17 | GCAATGAGCCTCAC CTTCCA | SEQ ID NO: 60 | AACCGATCAGCTGG GTATGT | SEQ ID NO: 61 |
| POT-18 | GGAACGCTGCCTGG AGAAAT | SEQ ID NO: 62 | CCCACAGCACAGCA GAAATT | SEQ ID NO: 63 |
| POT-19 | TCTTCTGCTCCATCC CACCTT | SEQ ID NO: 64 | GCATGAGCTTTGCC ACCAAA | SEQ ID NO: 65 |
| POT-20 | GCACAACGTAGCGC AAATCT | SEQ ID NO: 66 | GGGCCTGTTCTTCA AGACCAA | SEQ ID NO: 67 |
| POT-21 | GCCTGCCCTGCATT TTTCAA | SEQ ID NO: 68 | AAGGGGACAAGTG CTATGCA | SEQ ID NO: 69 |
| POT-22 | GCTGCAGGCAAATG GAGTTG | SEQ ID NO: 70 | CATGGGAGGAGCCT TAGCAA | SEQ ID NO: 71 |
| POT-23 | AGGGAGTAGCAAGT CTGGTCTT | SEQ ID NO: 72 | AGTGATTACCTGCC CCTCCTA | SEQ ID NO: 73 |
| POT-24 | CACCACGCCTGGCT CATTTA | SEQ ID NO: 74 | TTTCCTGTCAAAAA CCAACTGTTAGG | SEQ ID NO: 75 |
| POT-25 | AGCATACAGTAGGA AGTCCACAA | SEQ ID NO: 76 | GTTGGTCCCTGTGA TGAAAGC | SEQ ID NO: 77 |
| POT-26 | GCATCTCCCTGGTG ACGAAA | SEQ ID NO: 78 | CCAGTGGCTGTGGG ATTACT | SEQ ID NO: 79 |
| POT-27 | TCTCCTCCGAATGG CCTCAT | SEQ ID NO: 80 | TGATAGAGGCCACA GCTGAAG | SEQ ID NO: 81 |
| POT-28 | GGACGATGCCTGGA GTCTCT | SEQ ID NO: 82 | TGTCTCAGGGACTT ACCAACTTG | SEQ ID NO: 83 |
| POT-29 | ACACAGTGGCTTGT TCTCGAT | SEQ ID NO: 84 | TGCTTTCTGACTCC ACTTTTGT | SEQ ID NO: 85 |
| POT-30 | GGGGTGGACGCAT TGTTT | SEQ ID NO: 86 | TCACCCAGGGTCAC AGGATA | SEQ ID NO: 87 |
| POT-31 | TCCATTGACAGACA CTTCAGTTGT | SEQ ID NO: 88 | TGGTGCAGCCTCTG TGAAAA | SEQ ID NO: 89 |
| POT-32 | AGTGTCATGGGGCT TTTGTCT | SEQ ID NO: 90 | ACTTCCACGCTCCT CCTGAT | SEQ ID NO: 91 |
| POT-33 | TCCTTTGTTTCTGTT TGTGTGTGT | SEQ ID NO: 92 | TGCTGGGGGAAAA ACATCCA | SEQ ID NO: 93 |
| POT-34 | CTCAGGCTGCAGCA GAAATG | SEQ ID NO: 94 | AGGGAGCTGTTAGG TGCTTT | SEQ ID NO: 95 |
| POT-35 | ATGGCTGCCGTTTG CTCTAT | SEQ ID NO: 96 | TGGTGAAGCCCCTA TGACAAC | SEQ ID NO: 97 |
| POT-36 | GCACCGTGCATCAA AATTCA | SEQ ID NO: 98 | TGGAGACATTAATA CCTCACAGTGT | SEQ ID NO: 99 |
| POT-37 | CCCACCGAAACAAA CAATGTCA | SEQ ID NO: 100 | CTGCTTGTTTTCTTC CCCTTCA | SEQ ID NO: 101 |
| POT-38 | TTGTGTCCCAGAGC TCACTGT | SEQ ID NO: 102 | AAAATGCCTCGCAC CTGGTA | SEQ ID NO: 103 |
| POT-39 | TGCTTTTATCCCAG GCCTCCTA | SEQ ID NO: 104 | TGCACTCCATCCTG GTGACA | SEQ ID NO: 105 |
| POT-40 | ACACCATGCCACAC CATGAA | SEQ ID NO: 106 | TGGTCCCCCTGGAA ATGGA | SEQ ID NO: 107 |
| POT-41 | GCAACAGCTACGGA GGACTT | SEQ ID NO: 108 | AGGCAGCCCTTCAT AGAGTT | SEQ ID NO: 109 |
| POT-42 | GCCAGATGCCCTCC AAAGAG | SEQ ID NO: 110 | AGGTCATTTACAGA GCAGGCAAT | SEQ ID NO: 111 |
| POT-43 | TGGATTTCTAGTGA GAGCTGTTGA | SEQ ID NO: 112 | CAGTTGTTTTATCA CTGGACAAGAA | SEQ ID NO: 113 |
| POT-44 | ACTCCTCCAACCCC ATCATCT | SEQ ID NO: 114 | TGGGACATCGGAAC AGCTTT | SEQ ID NO: 115 |
| POT-45 | CACTTAGTGAACCG CAAACACA | SEQ ID NO: 116 | GCACTGCACACTTT TCAGACA | SEQ ID NO: 117 |

The primers are synthesized; the length of the amplified fragments is controlled at 200-250 bp; and a potential off-target region located in the middle. A sample of hematopoietic stem cells before gene editing serves as control; the completely produced sample serves as an experimental group; and the genomes are respectively extracted for amplification. Each amplification reaction comprises 100 ng of genome DNA (15000 cells). The obtained amplification product is used for constructing an amplification sublibrary in a PCR-free manner by using the NEBNext Ultra II kit; sequencing is conducted in a PE150 manner by an Illumina HiSeq X-ten sequencer; low-quality linker-containing data are filtered from download data by Fastp; the obtained clean data are subjected to two-end data jointing by Vsearch; the obtained high-quality sequence is aligned with an original targeted sequence and a sgRNA-PAM sequence in a blastn local alignment manner; and during subsequent analysis, the editing efficiency is calculated in manners that consistency with the targeted sequence is 40% or higher, a complete sgRNA-PAM region does not comprise any indel sequence as non- editing sequence and the sgRNA-PAM is incomplete to be editing sequence.The limit of detection accuracy of the NGS is 0.1%. Due to existence of sequencing errors and different degrees of the sequencing errors at different sites, the off-target sites are defined as follows: the editing efficiency of the sites in the editing group exceeds 0.1% and has significant difference from that in the control group (the gene editing efficiency is more than 2 times of that of the control group).

Through deep sequencing analysis, the gene editing efficiency of the 45 potential off-target sites and the target site is shown as Fig. 1.

In example 5, a method for combining PCR specific fragment amplification with deep sequencing is used in the editing group and the control group; and the potential off-target effect in the product is detected by using a specially constructed potential off-target recognition tag for the sgRNA. At the target sites, a specific gene editing efficiency of 74.39% is tested in the editing group and is far more than 0.05% in the Mock group. At the 45 potential off-target sites, the gene editing efficiency of the editing group has no significant difference from that of the Mock group; and most of the efficiency is about equal to the own limit of the detection (%)of the NGS or even lower. These data indicate that the sgRNA has excellent gene editing specificity; and the off-target effect is decreased to be minimum and is lower than the limit of the detection of the NGS.

## Claims

1. A method for determining suitability of a cell therapy for an individual, comprising administering a genetically modified cell population from an individual to the individual, wherein the genetically modified cells are modified at a target site through gene editing;
the method comprises the following steps:
a) determining occurrence of gene editing of each site in multiple off-target tagged sites in the genetically modified cells or offspring thereof;
b) assessing the suitability of the therapy based on the occurrence of gene editing;
wherein the nonoccurrence of gene editing at any of the multiple off-target tagged sites indicates that the cell therapy is suitable for the individual.

2. The method according to claim 1, wherein the determination step is conducted on the genetically modified cell before administering the genetically modified cells.

3. The method according to claim 2, further comprising: administering the genetically modified cells to the individual.

4. The method according to claim 1, wherein the determination step is conducted on the offspring of the genetically modified cell after administering the genetically modified cells.

5. The method according to claim 4, wherein the determination step is conducted within about at least one month after the administration of the genetically modified cells.

6. The method according to claim 4 or 5, further comprising repetition of the determination and assessment steps once or more times.

7. The method according to claim 6, wherein the determination and assessment steps are repeated at a frequency of about once every month to about once every year.

8. The method according to claim 7, wherein if sequence variation of the off-target tagged sites is less than 0.1%, or the sequence variation of the off-target tagged sites is less than 2 times compared with that of control cells that are not subjected to gene editing modification, the determination and assessment steps are repeated at a frequency of once every 3-12 months; and if sequence variation of the off-target tagged sites is greater than 0.1%, or the sequence variation of the off-target tagged sites is greater than 2 times compared with that of control cells that are not subjected to gene editing modification, the repetition frequency of the determination and assessment steps is increased with increase of the sequence variation frequency.

9. The method according to any of claims 5-8, wherein the method further comprises the treatment of the individual by an intervention therapy after the assessment step.

10. The method according to claim 9, wherein the intervention therapy comprises removal of a genetically modified cell population from an individual administered to the individual.

11. The method according to claim 9 or 10, wherein the intervention therapy comprises the administration of a second genetically modified cell population from the individual.

12. The method according to any of claims 1-11, wherein the multiple off-target tagged sites include at least about 10 off-target tagged sites.

13. The method according to any of claims 1-12, wherein sequence variation of less than 0.1% occurs at the off-target tagged sites, or compared with that of control cells that are not subjected to gene editing modification, sequence variation of less than 2 times occurs at the off-target tagged sites, which indicates that no gene editing occurs at the off-target tagged sites.

14. The method according to any of claims 1-13, wherein the determination step is conducted through DNA sequencing.

15. The method according to claim 14, wherein the determination step comprises: 1) amplifying nucleic acids comprising the multiple off-target tagged sites by using multiple primer sets; and 2) conducting sequencing analysis on the amplified nucleic acids.

16. The method according to any of claims 1-15, wherein the in-vitro test method comprises any one or more of the followings: BLESS, GUIDE-seq, HIGTS, Circle-seq, SITE-seq and Digenome-seq.

17. The method according to any of claims 1-16, wherein the in-vitro test method is conducted under a saturation condition.

18. The method according to any of claims 1-17, wherein the saturation condition allows effective cutting in at least more than 90% of the target sites.

19. The method according to claim 18, wherein the saturation condition allows effective cutting in 100% of the target sites.

20. The method according to any of claims 1-19, wherein the genetically modified cells are modified by the CRISPR/Cas system.

21. The method according to any of claims 1-20, wherein the target sites are located at BCL11A gene loci.

22. The method according to claim 21, wherein the off-target tagged sites refer to one or more of the sites as shown in Table 2.

23. A method for assessing off-target gene editing in a genetically modified cell population or offspring thereof, wherein the genetically modified cells are modified at the target sites through gene editing; the method comprises determination of the occurrence of gene editing of each site in the multiple off-target tagged sites; the multiple off-target tagged sites are obtained by manners as follows: 1) identifying first multiple off-target tagged sites based on their sequence similarity with the target sites; and/or 2) identifying second multiple off-target tagged sites by using an in-vitro test method; wherein sequence variation of more than 0.1% occurs at the off-target tagged sites, or compared with that of control cells that are not subjected to gene editing modification, sequence variation of more than 2 times occurs at the off-target tagged sites, which indicates that off-target gene editing occurs in the genetically modified cell population or offspring thereof.

24. The method according to claim 23, wherein the determination step is conducted through DNA sequencing.

25. The method according to claim 24, wherein the determination step comprises: 1) amplifying nucleic acids comprising the multiple off-target tagged sites by using multiple primer sets; and 2) conducting sequencing analysis on the amplified nucleic acids.

26. A method for obtaining multiple off-target tagged sites in a genetically modified cell population, wherein the genetically modified cells are modified at the target sites through gene editing; the method for obtaining the off-target tagged sites comprises: 1) identifying first multiple off-target tagged sites based on their sequence similarity to the target sites; and/or 2) identifying second multiple off-target tagged sites by using an in-vitro test method.

27. The method according to any of claims 23-26, wherein the in-vitro test methods comprise any one or more of the followings: BLESS, GUIDE-seq, HIGTS, Circle-seq, SITE-seq and Digenome-seq.

28. The method according to any of claims 23-27, wherein the in-vitro test method is conducted under a saturation condition.

29. The method according to claim 28, wherein the saturation condition allows effective cutting in more than 90% of the target sites.

30. The method according to any of claims 23-29, wherein the genetically modified cells are modified by the CRISPR/Cas system.

31. A kit for assessing off-target editing of a genetically modified cell population or offspring thereof, wherein the genetically modified cells are modified at the target sites through gene editing ; and the kit comprises: 1) one or more components of a gene editing system, used for producing the genetically modified cell; and 2) multiple primer sets used for amplifying nucleic acids comprising the multiple off-target tagged sites.

32. The kit according to claim 31, wherein the multiple off-target tagged sites are obtained by manners as follows:
1) identifying first multiple off-target tagged sites based on their sequence similarity with the target sites; and/or
2) identifying second multiple off-target tagged sites by using an in-vitro test method.

33. The kit according to any of claims 31-32, wherein the gene editing system is the CRISPR/Cas9 system.

34. The kit according to any of claims 31-33, further comprising one or more primer sets, wherein the primer sets are used for amplifying nucleic acids comprising the target sites.

35. The kit according to claim 34, wherein the one or more primer sets comprise one or more of the followings: SEQ ID NOs: 26-117.
